# FASCICULE DE BREVET EUROPEEN

(11) **EP 0 549 441 B1**
(45) Date de publication et mention de la délivrance du brevet: **06.08.1997**
(21) Numéro de dépôt: 92403478.8
(22) Date de dépôt: 21.12.1992
(51) Int. Cl.: A61K 31/44, A61K 31/495, A61K 31/50, A61K 31/505, A61K 31/53, A61K 31/425, A61K 31/54, A61K 31/71

(54) **Agents systémiques d'hormones juvéniles contre les ectoparasites**
Systemisch gegen Ektoparasiten anwendbare Juvenilhormone
Juvenile hormone agents for systemically treating ectoparasites

(30) Priorité: 23.12.1991 US 812430
(43) Date de publication de la demande: 30.06.1993
(73) Titulaire: Virbac, Inc., Fort Worth, Texas 76137 (US)
(72) Inventeur: Miller, Thomas A., Forth Worth, Texas 76118-6904 (US)
(74) Mandataire: Orès, Bernard

(56) Documents cités:
- EP-A- 0 214 731
- EP-A- 0 255 803
- GB-A- 2 140 010
- CHEMICAL ABSTRACTS, vol. 110, 1989, page 236, résumé no. 2529t, Columbus, Ohio, US; T. SEGAWA et al.: "General pharmacology of 2-[1-methyl-2-(4-phenoxyphenoxy)ethoxy]pyridine (S-31183)"

## Description

La présente invention est relative à certains composés hétérocycliques contenant de l'azote pour la lutte systémique contre les ectoparasites sur les animaux homoiothermiques ou à sang chaud et par ailleurs, à l'utilisaiton de combinaisons des composés hétérocycliques contenant de l'azote avec d'autres ingrédients actifs par voie systémique pour lutter à la fois contre les ectoparasites et les endoparasites.

Les ectoparasites suceurs de sang de l'ordre Insecta comprennent, par exemple, *Ctenocephalides felis* et *Ctenocephalides canis* (puces des chats et des chiens), ainsi que les poux, les moustiques, les tabanidés, les mouches tsé-tsé et d'autres mouches qui piquent, et Acarina comme *Boophilus, Amblyomma, Anocentor, Dermacentor, Haemaphysalis, Hyalomma, Ixodes, Rhipicentor, Margaropus, Rhipicephalus, Argas, Otobius* et *Ornithodoros* (tiques) et similaires, infestent ou attaquent de nombreux animaux homoiothermiques utiles comprenant les animaux d'élevage comme le bétail, les porcs, les moutons, les chèvres, la volaille telle que poulets, dindes et oies, les animaux à fourrure comme le vison, les renards, le chinchilla, les lapins et similaires, et les animaux familiers comme les chiens et les chats.

Les tiques comprennent les tiques dures ou tiques tendres et sont caractérisées en tant que tiques d'un hôte, de deux hôtes ou de trois hôtes. Elles s'attaquent à un animal hôte convenable et se nourrissent de son sang et de ses fluides corporels. Les femelles gonflées de sang se détachent et tombent de l'hôte et pondent de grands nombres d'oeufs (2.000 à 20.000) dans une niche convenable dans le sol ou dans un autre endroit abrité où a lieu l'éclosion. Les larves cherchent ensuite un hôte susceptible de l'alimenter en sang. Les larves des tiques à un hôte vont une deuxième fois sur l'hôte pour devenir des nymphes et des adultes sans quitter l'hôte. Les larves des tiques à deux ou trois hôtes tombent de l'hôte, vont dans l'environnement et trouvent un deuxième ou un troisième hôte (en tant que nymple ou adulte) sur lequel elles se nourrissent.

Les tiques sont responsables de la transmission et de la propagation de nombreuses maladies humaines et animales dans le monde entier. Les tiques d'importance économique majeure comprennent *Boophilus, Rhipicephalus, Ixodes, Hyalomma, Amblyomma* et *Dermacentor.* Elles sont les vecteurs de maladies bactériennes, virales, à rickettsies et protozoaires et provoquent la paralysie de la tique et la toxicose de la tique. Même une seule tique peut provoquer une paralysie à la suite de l'injection de sa salive dans l'hôte pendant qu'elle s'alimente. Les maladies portées par les tiques sont généralement transmises par des tiques à hôtes multiples. Ces maladies, telles que babésiose, anaplasmose, theilériose et filariose sont responsables de la mort et/ou de la débilitation de grands nombres d'animaux familiers et des animaux à viande dans le monde entier. Dans de nombreuses régions tempérées, les tiques Ixodid transmettent l'agent d'une maladie chronique débilitante, la maladie de Lyme, de la vie sauvage à l'homme. En plus de la transmission des maladies, les tiques sont responsables de grandes pertes économiques dans la production des animaux sur pied. Les pertes sont attribuables non seulement à la mort, mais aussi au fait que les peaux sont abîmées, à la perte de croissance, à la réduction de la production laitière et à la moindre qualité de la viande. Bien que les effets débilitants des infestations de tiques sur les animaux aient été reconnus depuis des années et que des progrès énormes aient été faits dans les programmes de lutte contre les tiques, il n'existe aucune méthode entièrement satisfaisante pour lutter contre ces parasites ou les éradiquer, et les tiques ont souvent développé une résistance aux produits toxiques chimiques et aux mesures de lutte qui en dépendent.

L'infestation des animaux familiers par les puces a été longtemps une nuisance pour les propriétaires de ces animaux. Comme les puces sont capables de survivre et de se multiplier dans une large gamme de conditions environnementales, la lutte contre l'infestation par les puces requiert un programme à plusieurs facettes qui doit être rigoureusement appliqué pour atteindre un certain succès. Les puces adultes vivent dans les poils du chien ou du chat et se nourrissent de sang. Les puces mâles et femelles s'apparient encore dans les poils de l'animal et la puce femelle y pond ses oeufs. Les oeufs n'adhèrent pas aux poils, mais tombent et sont distribués dans l'environnement de l'animal. Par ce mécanisme, tandis que l'environnement total de l'animal familier est infesté avec les oeufs de puce, l'infestation est le plus forte dans les sites dans lesquels les animaux passent le plus de temps. Les oeufs éclosent en larves en environ 2 jours. Il y a trois stades larvaires, durant chacun environ trois jours. Dans le dernier stade, les larves filent un cocon et se transforment en nymphes. Dans les conditions optimales, (c'est-à-dire 33°C et 65% d'humidité relative), les oeufs se développent depuis les larves aux nymphes en environ 8-10 jours. Après une période supplémentaire d'environ 8 jours, les nymphes se développent en jeunes puces adultes dans le cocon et sont encore dispersées dans l'environnement de l'animal familier. Ces puces adultes pré-émergées attendent dans leur pupes jusqu'au moment où elles détectent, par la tension de dioxyde de carbone et/ou des vibrations, la présence d'un hôte animal et elles sortent alors brutalement et sautent dans l'air sur l'hôte qui passe. Dans des conditions environnementales appropriées de température et d'humidité, les puces émergées non nourries qui ne réussissent pas à trouver un hôte peuvent survivre pendant un certain temps dans l'environnement, dans l'attente d'un hôte convenable. Il faut au moins trois semaines pour que des oeufs se développent en adultes pré-émergés, capables de réinfester un animal hôte. Cependant, les adultes pré-émergés peuvent rester viables dans le cocon pendant des mois, voire une année. De plus, dans des conditions de températures sub-optimales, il peut falloir 4-5 mois pour que les oeufs se développent en pupes contenant les adultes pré-émergés. Les puces doivent être alimentées avec du sang pour devenir sexuellement matures et capables de se reproduire. Après leur premier repas de sang, elles subissent un changement de métabolisme tel qu'elles ne peuvent pas survivre pendant un temps quelconque hors de l'hôte. Le sang doit provenir de l'animal correct et l'appétit de la puce femelle nécessite qu'elle consomme jusqu'à 5 fois son poids corporel de sang chaque jour. Le long cycle de vie et en particulier, la période prolongée de dormance en pré-émergence, a rendu la lutte contre les puces avec des composés appliqués topiquement à des animaux domestiques difficile et pas entièrement satisfaisante. La plupart des composants actifs appliqués topiquement ont un effet résiduel limité, si bien que la réinfestation par les adultes nouvellement émergés provenant de l'environnement de l'animal familier est un problème constant.

L'infestation des chiens et des chats avec les puces a plusieurs effets indésirables pour les animaux et leurs propriétaires. Ces effets indésirables comprennent une irritation locale et des démangeaisons désagréables conduisant à un grattage. Une forte proportion d'animaux familiers, en particulier les chiens, deviennent allergiques à la salive de puce, aboutissant à l'état chronique connu sous le nom d'allergie à la morsure de puce (ou allergie aux puces). Cet état incite l'animal à se mordre et à se gratter, ce qui conduit à une excoriation de la peau, une infection pyogène secondaire, une perte de poils et à de graves changements cutanés inflammatoires chroniques. De plus, la plupart des chiens et des chats qui sont infestés par les puces deviennent aussi infectés avec *Dipylidium caninum*, le ténia transmis par les puces.

En l'absence prolongée d'un animal convenable, les puces nouvellement émergées s'attaquent aux mammifères, y compris l'homme, quoiqu'elles ne puissent pas disposer de leur potentiel reproductif complet si le sang humain est leur seule source de nutrition. Même en présence de l'animal domestique, le propriétaire peut être piqué par des puces, Certains hommes peuvent souffrir de maladies de peau allergiques après avoir été mordus par des puces de chiens et de chats.

Comme, de même que la plupart des insectes, les puces peuvent s'adapter pour survivre à une exposition à des agents normalement toxiques et que la tolérance des chiens et des chats à des agents chimiques varie, il est souhaitable de disposer d'un grand nombre d'agents et de méthodes pour lutter contre les puces. Les méthodes de l'art antérieur comprennent de nombreux agents toxiques, comme les organophosphates (par exemple, le chlorpyrifos), des carbamates (par exemple, le Carbaryl), des pyréthroïdes (par exemple, des pyréthrines et la perméthrine), et d'autres insecticides topiques formulés et conçus pour tuer la puce adulte après leur application sur l'animal familier. Beaucoup des agents toxiques ayant une action résiduelle efficace contre les puces, comme le DDT, l'hexachlorure de benzène et d'autres insecticides de type hydrocarbure chloré, ont été interdits dans la plupart des pays en raison de la persistance de résidus dans l'environnement et de leur effet sur une certaine vie sauvage. D'autres ont été interdits en raison des risques pour la santé à long terme, y compris des risques de cancer pour les humains exposés de façon chronique. Aux USA, même des agents toxiques couramment autorisés et disponibles qui sont efficaces contre les puces, certains seulement brièvement, sont soumis à des examens minutieux pour déterminer les risques sur la santé à long terme pour les animaux familiers et leur propriétaire. Ces considérations ont limité l'utilité des composés toxiques insecticides et acaricides dans la lutte contre les puces et les tiques sur les animaux familiers et les ectoparasites sur les animaux en général.

En plus des nombreuses compositions insecticides visant à lutter contre les ectoparasites, des agents systémiques dirigés contre des ectoparasites ont été suggérés. Par exemple, les brevets US 3.962.458 et 4.031.239 décrivent l'application de divers composés de type cyclopropane carboxylate (pyréthroïdes) pour lutter contre des ectoparasites par traitement systémique des animaux à sang chaud. Le brevet US 4.006.236 décrit l'utilisation systémique d'octahydrophénanthridines substituées. Le brevet US 4.053.631 décrit la lutte par voie systémique d'ectoparasites avec des alpha-cyano-m-phénoxybenzylalpha-alkyl (en C₁₋₄)-2-naphtalèneacétates. Le brevet US 4.323.582 décrit l'utilisation systémique d'alcanolamines inférieures pour repousser les parasites suceurs de sang.

Les brevets US 4.089.975 et 4.092.421 décrivent l'administration par l'alimentation de certains composés hétérocycliques contenant de l'azote pour lutter contre les insectes se reproduisant dans le fumier, par administration orale d'une quantité efficace du point de vue insecticide du composé à un animal à sang chaud. Ces applications impliquent un mécanisme par l'alimentation dans lequel l'ingrédient actif est administré par voie orale à l'animal hôte, traverse son système digestif sans être métabolisé et est excrété sous une forme active du point de vue insecticide. Ce mécanisme implique une médiocre absorption de ces composés et ne suggèrent pas que ces composés hétérocycliques contenant de l'azote passent dans le flux sanguin de l'animal et circulent dans le sang sous une forme active.

L'utilisation en tant qu'agents systémiques des classes d'hormones juvéniles ressemblant au méthoprène, des dérivés de benzoylurée inhibant la croissance des puces et des dérivés de triazine inhibant la croissance des puces, est décrite dans le brevet US 4.973.589. Une activité ovicide est montrée pour certains dérivés de la triazine, ainsi que pour les dérivés de la benzoylurée dans le brevet US 4.973.589, mais aucun exemple n'est donné pour la classe d'hormones juvéniles. Une activité ovicide chez une espèce cible a été décrite pour les matières de type hormone juvénile, mais seulement lorsqu'elles étaient appliquées topiquement à l'animal.

En raison de la variabilité des effets toxiques chez les diverses espèces animales et des niveaux de doses élevés requis pour obtenir des effets systémiques avec beaucoup des composés de l'art antérieur, il est souhaitable de disposer d'autres agents de lutte supplémentaires. Certains composés hétérocycliques substitués d'activité insecticide connue sont décrits dans les brevets US 4.970.222, 4.879.292 et 4.751.223. Cependant, ces composés hétérocycliques contenant de l'azote de type hormone juvénile n'ont pas été suggérés jusqu'à maintenant en tant qu'agents ovicides administrés de façon systémique, selon laquelle une dose efficace du point de vue ovicide est administrée a l'ectoparasite cible lorsqu'il se nourrit du sang de l'animal traité. Des travaux rapportés dans CHEMICAL ABSTRACTS (Résumé n° 2529t, vol. 110, 1989, 236) ont même montré que le pyriproxifen qui représente le chef de file de ces composés hétérocycliques présentent une activité pharmacologique très faible, voire nulle chez les mammifères.

Dans de nombreuses régions du monde, les conditions de l'environnement et les méthodes utilisées pour maintenir les populations animales qui favorisent la multiplication et la survie des ectoparasites, encouragent de façon analogue la prolifération et la dissémination des endoparasites, a la fois ceux qui ont un cycle de vie vivante libre (par exemple, les parasites de type ascaride des intestins de l'ordre *Nematoda*) et ceux qui dépendent de la transmission par des vecteurs (par exemple, parasites du même ordre habitant dans les poumons, les courants lymphatique et sanguin vasculaire). Des exemples de parasites intestinaux qui fleurissent dans ces conditions favorables sont les ascarides et les ankylostomes des chiens et des chats. Les parasites intestinaux du même ordre posent aussi un problème majeur pour l'industrie animale alimentaire et de récréation, parasitant le bétail, les moutons, les porcs et les chevaux. Un exemple d'un nématode parasite interne transmis par un vecteur est le filaire cardio-pulmonaire du chien et dans une moindre mesure du chat, transmis par les moustiques. En raison des conditions requises pour les conditions environnementales et les méthodes analogues de gestion des animaux, il est extrêmement courant que les animaux de compagnie et les animaux de rapport soient en même temps exposés et infectés par des endoparasites et infestés par des ectoparasines comme les puces, les tiques, les poux, les acariens de la gale, les mouches qui piquent, y compris les moustiques, qui est le vecteur d'un très important endoparasite, le filaire cardio-pulmonaire du chien. On a en conséquence énormément besoin de composés susceptibles d'empêcher et de traiter à la fois les infectations par des endoparasites et des infestations par des ectoparasites.

On a maintenant trouvé de façon surprenante, que la classe des composés hétérocycliques de type hormone juvénile. qui sont décrits dans les brevets US 4.970.222, 4.879.292 et 4.751.223, et représentés par la formule générale : 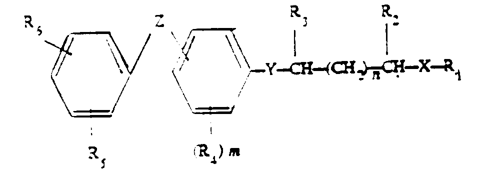 dans laquelle R₁ est l'un des groupes suivants : 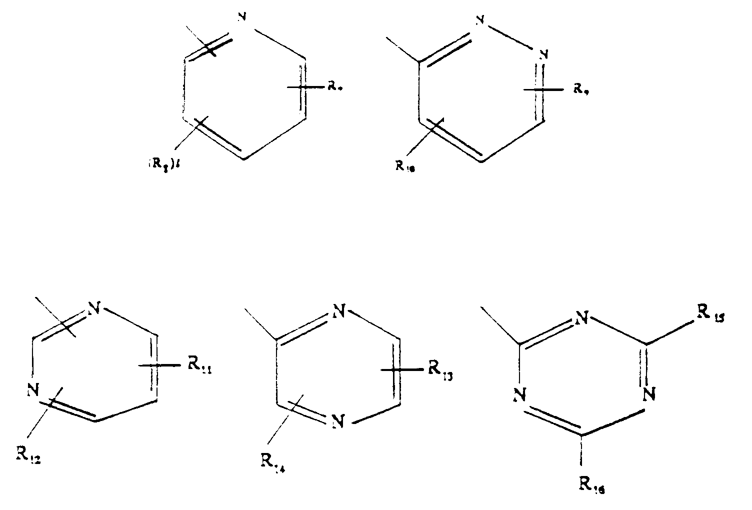 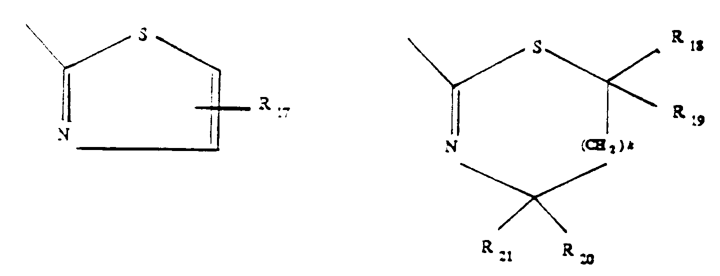 dans lesquels R₇, R₈, R₉, R₁₀, R₁₁, R₁₂, R₁₃, R₁₄, R₁₅, R₁₆ et R₁₇, identiques ou différents, sont chacun un atome d'hydrogène ou d'halogène, un groupe alcoxy en C₁₋₄, alkylthio en C₁₋₄, trifluorométhyle ou nitro; R₁₈, R₁₉, R₂₀ et R₂₁, identiques ou différents, sont chacun un atome d'hydrogène ou un groupe méthyle, *k* est un entier de 0 à 1, et *l* est un entier de 0 à 3; R₂ et R₃, identiques ou différents, sont chaucun un atome d'hydrogène ou d'halogène ou un groupe méthyle; R₄ est un atome d'halogène ou un groupe méthyle; R₅ et R₆, identiques ou différents, sont chacun un atome d'hydrogène ou d'halogène, un groupe haloalkyle en C₁₋₄, ou haloalcoxy en C₁₋₄; X, Y et Z, identiques ou différents, sont chacun un atome d'oxygène, de soufre ou un groupe méthylène, *m* est un entier de 0 a 4, et *n* est un entier de 0 à 2, comprenant en particulier le pyriproxifen, la 2-[1 -méthyl-2-(4-phénoxyphénoxy)-éthoxy]-pyridine (disponible dans le commerce chez Sumitomo Chemical Company ou chez McLaughlin Gormley, King Co. sous la dénomination commerciale NYLAR®), sont efficaces par voie systémique pour lutter contre les ectoparasites chez les animaux homoiothermiques (à sang chaud). Lorsque ces composés sont administrés de façon systémique à de très faibles doses à des animaux d'essai, ils présentent un puissant effet ovicide à l'encontre des ectoparasites. Le terme ectoparasite utilisé ici, a sa signification normale dans l'art et englobe les puces, les tiques, les poux, les moustiques, les tabanidés, les mouches tsé-tsé et autres mouches qui piquent et en particulier, les espèces nommées plus haut.

La présente Invention a donc pour objet l'utilisation d'un composé hétérocyclique du type hormone juvénile décrit ci-avant pour la préparation d'une composition pharmaceutique destinée à la lutte systémique des ectoparasites qui attaquent les animaux à sang chaud, telle que définie dans les Revendications 1 à 8.

La présente Invention a également pour objet une composition comprenant une quantité efficace du point de vue ovicide d'un tel composé et une quantité du point de vue parasiticide d'un composé choisi dans le groupe consistant en avermectine, dérivés d'avermectine, milbémycine, dérivés de milbémycine, ivermectine, dérivés d'ivermectine, milbémycine oxime, dérivés de milbémycine oxime, moxidectine et dérivés de moxidectine, ou leurs mélanges, telle que définie dans les Revendications 9 à 15.

La présente Invention a encore pour objet l'utilisation d'une telle composition pour la préparation d'une composition pharmaceutique pour la lutte systémique contre les parasites, telle que définie dans les Revendications 16 à 18.

La présente Invention a également pour objet l'utilisation d'une telle composition pour la préparation d'une composition pharmaceutique pour la lutte systémique simultanée contre les ectoparasites et les endoparasites chez les animaux à sang chaud, telle que définie dans les Revendications 19 à 22.

La présente Invention a enfin pour objet un supplément alimentaire comprenant une telle composition, tel que défini dans les Revendications 23 à 27.

Les ingrédients actifs systémiquement efficaces contre plusieurs des endoparasites comprennent les avermectines, les dérivés de type avermectine et un groupe d'antibiotiques apparentés, tels que par exemple. milbémycine, dérivés de type milbémycine, ivermectine, dérivés de type ivermectine, milbémycine oxime, dérivés de type milbémycine oxime, moxidectine, dérivés de type moxidectine et leurs mélanges, tels que décrits dans les brevets US 4.346.177 et 3.950.360 (milbémycine), 4.199.569 (ivermectine), 4.547.520 (milbémycine oxime) et 4.988.824 et les demandes incorporées (moxidectine).

Les infections par le filaire cardio-pulmonaire, les ascarides et les ankylostomes des chiens et des chats peuvent être prévenues par administration des composés antibiotiques de type avermectine par injection parentérale, administration orale, application transdermique et par implantation de dispositifs solides ou creux conçus pour libérer de façon contrôlée et prolongée le ingrédient actif. Par exemple, la milbémycine, l'ivermectine, la milbémycine oxime et la moxidectine peuvent être administrées chaque mois pour prévenir et guérir les infections par le filaire cardio-pulmonaire, les ascarides et les ankylostomes des chiens et des chats.

Il est particulièrement commode pour empêcher une infection et une maladie provoquées par ces endoparasites et pour lutter contre l'infestation ectoparasite, d'utiliser la combinaison d'un produit unique d'un composé hétérocyclique contenant de l'azote actif par voie systémique de l'invention, tel que défini plus haut et de l'un des composés de la classe des antibiotiques efficaces contre les endoparasites (par exemple, incluant sans limitation, la milbémycine, l'ivermectine, la milbémycine oxime et la moxidectine). L'administration de cette combinaison sûre, efficace et commode peut être faite par traitement quotidien ou périodique (par exemple, mensuel) avec des formes de doses orales liquides, en tablettes à mâcher ou en comprimés, par injection parentérale, par une implantation moins fréquente d'un dispositif à libération contrôlée qui contient et libère à la fois un composé hétérocyclique contenant de l'azote actif de façon systémique de type hormone juvénile et un antibiotique actif de façon systémique efficace contre les parasites de type nématode à des taux qui fournissent et maintiennent des concentrations adéquates dans le sang pour affecter les endo- et ectoparasites cibles.

Conformément à la présente Invention, les ectoparasites sont exposés à une quantité efficace du point de vue ovicide de l'ingrédient actif lorsqu'ils ingèrent le sang d'un animal qui a été traité avec une dose efficace de façon systémique dans un support convenable. Les composés peuvent être administrés a des doses d'environ 0,0001 mg/kg à environ 1000 mg/kg, de préférence d'environ 0,01 mg/kg à environ 100 mg/kg, avantageusement d'environ 0,01 mg/kg à environ 70 mg/kg et le plus avantageusement d'environ 0,02 mg/kg à environ 50 mg/kg. Selon un mode de réalisation préféré, les composés utilisés en de telles quantités peuvent être combinés avec des agents de lutte contre les endoparasites, tels que par exemple, avermectine, dérivés de type avermectine, milbémycine, dérivés de type milbémycine, ivermectine, dérivés de type ivermectine, milbémycine oxime, dérivés de type milbémycine oxime, moxidectine, dérivés de type moxidectine et leurs mélanges, à des doses de l'ordre d'environ 0,5 µg/kg à environ 100 mg/kg. La préparation des ingrédients actifs contre les ectoparasites peut être faite selon les procédures décrites dans l'un quelconque des brevets US 4.920.222, 4.879.292 ou 4.751.223. Les antibiotiques luttant contre les endoparasites peuvent être préparés selon les procédures décrites dans les brevets US 4.346.171, 3.950.360, 4.199.569, 4.547.520 et 4.988.824 et les demandes incorporées. Le terme "dérivé" tel qu'il est utilisé ici, comprend des composés qui présentent des propriétés de lutte contre les endoparasites.

Une caractéristique essentielle de la présente invention est que l'ingrédient actif est administré de telle façon qu'il peut être ingéré par le parasite en train de se nourrir du sang de l'animal hôte, et peut ainsi manifester une activité contre les oeufs. Le terme "hôte" utilisé ici, entend un animal hôte dont le sang permet à un ectoparasite de développer ses capacités reproductrices normales. L'expression "efficace du point de vue ovicide", entend un effet qui conduit à un taux réduit d'éclosion des oeufs ou à l'incapacité du mâle à fertiliser les oeufs, aboutissant à une production d'oeufs stériles. Selon la présente invention, ceci est obtenu par plusieurs formes d'application, par exemple, par administration d'un ingrédient actif formulé par voie orale, parentérale, par implant ou en tant que bolus. Dans ces cas, le terme "formulé" signifie sous la forme d'une poudre, d'un comprimé, d'une gaufrette, d'un granulat, d'une gélule, d'une émulsion, d'un gel, d'une mousse, ou d'autres compositions convenables pour l'administration d'une quantité efficace de l'ingrédient actif. La préparation ne doit pas nécessairement être administrée directement à l'animal; il peut être commode de la mélanger à l'alimentation de l'animal. Les compositions peuvent contenir des adjuvants employés habituellement dans l'art de la formulation et les compositions à administrer par voie orale peuvent bien sûr contenir d'autres additifs qui stimulent l'ingestion volontaire par l'animal, comme des arômes ou des senteurs convenables. En raison de sa simplicité, l'application orale est l'un des modes préférés de la présente invention. Un autre mode d'application est la voie parentérale, par exemple, par injection sous-cutanée, intra-veineuse ou intra-musculaire, ou au moyen d'une préparation à action prolongée sous la forme d'un implant, d'un bolus ou d'une autre formulation à libération prolongée. L'application peut être sous forme de dose multiple ou de dose unique.

Les méthodes d'application orale comprennent, sans limitation, les composés prémélangés à l'alimentation animale, introduits dans des biscuits ou des friandises, des tablettes à mâcher ou des gaufrettes, des comprimés ou gélules à dissoudre, des concentrats émulsifiables, des composés solubles dans l'eau appliqués avec un compte-gouttes dans l'eau, ou des matières appliquées sous une forme quelconque sur les aliments pour animaux familiers. Les implants peuvent comprendre un quelconque dispositif appliqué à l'animal capable de libérer des composés pour lutter contre des ectoparasites. Il est envisagé que le produit de la présente invention puisse être aussi distribué à l'animal par un système de transport transdermique. L'administration percutanée est commodément accomplie par application sous-cutanée, dermique, intra-musculaire et même intra-veineuse de la formulation injectable. Des dispositifs classiques d'injection du type à aiguille, ainsi que des dispositifs d'injection à jet d'air sans aiguille, ainsi que des formulations à verser ou à étaler peuvent être utiles. Il est possible de retarder ou de prolonger la pénétration de l'ingrédient actif à travers les tissus vivants de l'animal par une formulation convenable.

L'action prolongée de l'ingrédient actif peut être obtenue par formulation du composé dans une matrice qui va inhiber physiquement la dissolution. La matrice formulée est injectée ou implantée chirurgalement d'une autre façon dans le corps, dans lequel elle reste sous forme d'un dépôt à partir duquel le composé se dissout lentement, ou dans le cas des composés hydrophobes, est libéré par diffusion. Les formulations de matrices connues maintenant dans l'art sont formulées dans des semi-solides cireux, comme des cires végétales et des polyéthylène glycols de haut poids moléculaire. Une action prolongée très efficace est obtenue par introduction dans l'animal d'un implant contenant l'ingrédient actif. Ces implants sont maintenant bien connus dans l'art vétérinaire et sont généralement faits de caoutchouc au silicium ou d'une autre matière plastique polymérisée comme un méthacrylate. Une composition d'implant particulièrement utile est décrite dans le brevet US 4.694.974. L'ingrédient actif est dispersé dans l'implant solide ou est contenu à l'intérieur d'un implant creux. L'ingrédient actif est dispersé par dissolution préalable ou mélange avec le polymère ou il est dissous dans un support ou mélangé à celui-ci et dispersé à l'intérieur du polymère. Après implantation, l'ingrédient actif diffuse ou est lessivé à partir de l'implant solide ou creux dans les fluides corporels de l'animal traité.

La vitesse à laquelle l'ingrédient actif est libéré à partir d'un implant et donc la période de temps pendant laquelle l'implant reste efficace, est contrôlée avec une bonne précision par l'ajustement convenable de la concentration du composé dans l'implant, la surface extérieure et la quantité du support dans l'implant, la surface extérieure de l'implant, la formulation du polymère à partir duquel est fait l'implant, l'épaisseur de la paroi des implants creux et les caractéristiques de diffusion de la solution de l'ingrédient actif ou du support/ingrédient actif à travers la paroi de l'implant ou à travers les bouchons terminaux conçus spécialement faits de polymère ou d'une autre membrane formant une ou plusieurs surfaces de l'implant, ou par passage forcé à travers une membrane poreuse ou une ouverture sous l'effet d'une pompe osmotique activée par l'absorption d'eau du corps dans un composant osmotiquement actif contenu dans un second compartiment d'un implant creux.

L'administration de l'ingrédient actif au moyen d'un implant est un autre mode de réalisation particulièrement préféré. Une telle administration est extrêmement économique et efficace parce qu'un implant convenablement conçu maintient une concentration constante du composé dans les tissus de l'animal hôte, peut être conçu pour libérer un composé pendant plusieurs mois et est facilement inséré dans l'animal. Aucune autre manipulation de l'animal ou aucun problème de dosage ne sont impliqués après l'insertion de l'implant. Cet implant peut être érodable/soluble et peut être laissé dans le tissu de l'animal, ou bien il peut être insoluble/non érodable et convenable pour une élimination chirurgicale après départ de son ingrédient actif.

Une formulation de bolus commode est décrite dans le brevet US 4.166.107, qui peut aussi être adoptée pour la libération prolongée pour la méthode de la présente invention.

La formulation d'additifs vétérinaires dans un aliment pour animaux est un art extrêmement bien connu. Il est usuel de formuler le composé d'abord sous forme de pré-mélange dans lequel l'ingrédient actif est dispersé dans un support liquide ou solide particulaire. Le pré-mélange peut commodément contenir d'environ 1 à environ 800 g de composé par kg, en fonction de la concentration désirée dans l'alimentation. Pour empêcher une hydrolyse ou une dégradation par des constituants de l'aliment pour animaux, le composé actif peut être formulé dans une matrice protectrice, comme de la gélatine, avant addition au pré-mélange, et davantage protégé par une formulation avec des conservateurs appropriés, et des anti-oxydants (par exemple, le benzoate de sodium, les parabens, le BHT (hydroxytoluène butylé) et le BHA (hydroxyanisole butylé).

La présente invention concerne aussi l'utilisation d'une quantité efficace du point de vue ovicide d'un composé de formule générale : 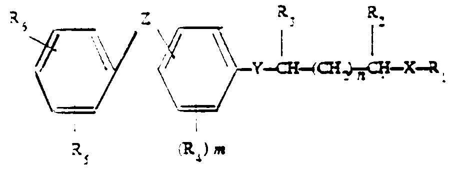 dans laquelle R₁ est l'un des groupes suivants : 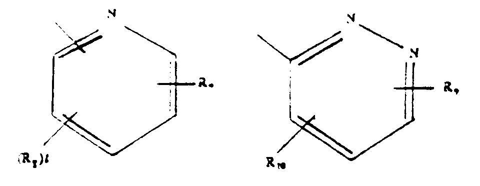 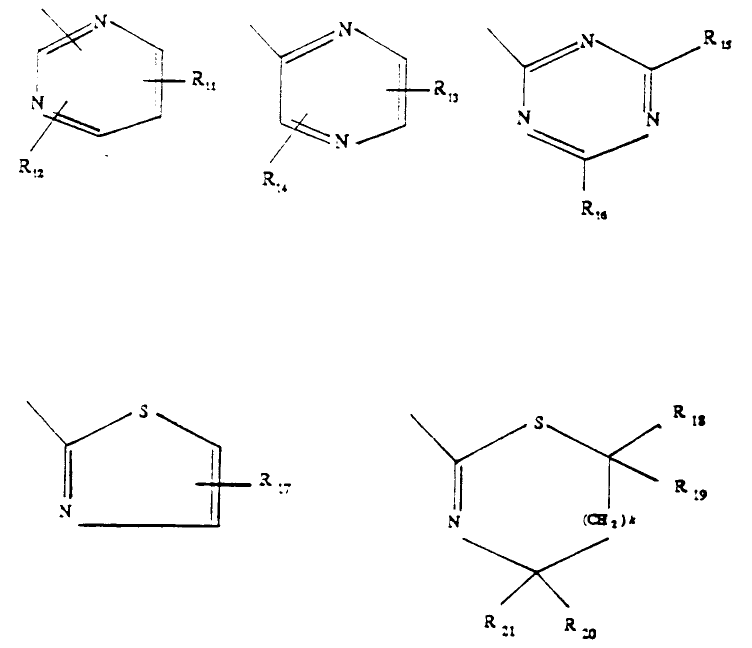 dans lesquels R₇, R₈, R₉, R₁₀, R₁₁, R₁₂, R₁₃, R₁₄, R₁₅, R₁₆ et R₁₇, identiques ou différents, sont chacun un atome d'hydrogène ou d'halogène, un groupe alcoxy en C₁₋₄, alkylthio en C₁₋₄, trifluorométhyle ou nitro; R₁₈, R₁₉, R₂₀ et R₂₁, identiques ou différents, sont chacun un atome d'hydrogène ou un groupe méthyle, *k* est un entier de 0 à 1, et *l* est un entier de 0 à 3; R₂ et R₃, identiques ou différents, sont chaucun un atome d'hydrogène ou d'halogène ou un groupe méthyle; R₄ est un atome d'halogène ou un groupe méthyle; R₅ et R₆, identiques ou différents, sont chacun un atome d'hydrogène ou d'halogène, un groupe haloalkyle en C₁₋₄, ou haloalcoxy en C₁₋₄; X, Y et Z, identiques ou différents, sont chacun un atome d'oxygène, de soufre ou un groupe méthylène, *m* est un entier de 0 à 4, et *n* est un entier de 0 à 2, qui est transmis à l'ectoparasite par le sang de l'animal pour la préparation d'une composition pharmaceutique administrable par voie orale, parentérale ou par implant et destinée a empêcher l'infestation des chiens et des chats par les puces.

Selon la présente invention, pour la prévention de la propagation des puces, on fournit aux puces un sang nutritif, qui contient une quantité efficace d'un ingrédient actif, et plus spéciftiquement, on alimente l'animal hôte avec une quantité efficace de l'ingrédient actif et on laisse les puces se nourrir sur celui-ci. Le composé est commodément appliqué par voie orale a une dose d'environ 0,01 à environ 200 mg/kg, de préférence d'environ 0,1 a environ 100 mg/kg et plus avantageusement d'environ 0,1 à environ 50 mg/kg de poids corporel, par rapport a l'animal hôte. Une bonne dose pour une administration régulière est en général de l'ordre d'environ 0,2 a environ 50 mg/kg de poids corporel de l'animal hôte. Les doses sont utilement répétées de façon régulière a des intervalles quotidiens ou mensuels. De fortes doses uniques peuvent être efficaces pendant une période même plus longue. Ces doses sont de préférence d'environ 1 à environ 1000 mg/kg, plus avantageusement d'environ 10 à 200 mg/kg et le plus avantageusement d'environ 20 à 100 mg/kg. La dose totale d'un ingrédient actif particulier peut varier d'un genre d'animal à l'autre et peut même varier dans le même genre, puisque la dose préférée dépend, entre autres choses, du poids et de la constitution de l'animal;

Conformément à la présente Invention, l'ingrédient actif n'est pas normalement appliqué sous une forme pure, mais de préférence sous la forme d'une composition qui, en plus de l'ingrédient actif, contient des activateurs d'application qui sont tolérés par l'animal hôte. Naturellement, en plus de la méthode de lutte contre les stades de développement juvénil selon la présente invention, les ectoparasites adultes peuvent aussi être contrôlés par des méthodes classiques, en particulier par application topique de produits toxiques insecticides et acaricides. Cependant, ces mesures supplémentaires ne sont pas absolument nécessaires. La composition à appliquer par la méthode de cette invention contient usuellement 0,1 a 99% en poids, de préférence 0,1 à 95% en poids de composé actif et 99,9 à 1% en poids, de préférence 99,9 à 5% en poids, d'un adjuvant non toxique solide ou liquide, et peut aussi comprendre de préférence au moins 0 à 25% en poids, par exemple 0,1 à 25% en poids d'un tensioactif non toxique.

Les produits commerciaux peuvent être formulés en concentrats, à partir desquels l'utilisateur final emploie normalement des formulations diluées. Les compositions peuvent aussi contenir d'autres composants comme des stabilisants, des antioxydants, des anti-mousses, des régulateurs de viscosité, des liants, des agents tackifiants, des conservateurs, ainsi que d'autres composants connus et actifs pour obtenir des effets spéciaux. Les matières connues dans la pratique vétérinaire comme étant convenables pour l'administration orale, parentérale ou par implant, peuvent être employées pour faciliter la formulation. Un certain nombre d'exemples sont cités ci-après.

On peut utiliser des supports convenables, en particulier des charges comme des sucres, le lactose, le sucrose, le manitol ou le sorbitol, des préparations cellulosiques et/ou des phosphates de calcium, par exemple, le phosphate tricalcique ou le phosphate acide de calcium, ainsi que des liants comme des pâtes d'amidon, utilisant par exemple, l'amidon de mais, de blé, de riz ou de pomme de terre, la gélatine, la gomme adragante, une méthylcellulose, et/ou si cela est désiré, des agents de désagrégation, comme les amidons mentionnés plus haut, ainsi qu'un carboxyméthyl amidon réticulé à une polyvinylpyrrolidone, un agar, l'acide alginique ou un sel de celui-ci, comme l'alginate de sodium. Les adjuvants sont des agents régulant l'écoulement et des lubrifiants, par exemple, la silice, le talc, l'acide stéarique ou ses sels, comme le stéarate de magnésium ou le stéarate de calcium et/ou un polypropylène glycol. Des noyaux de dragée peuvent être pourvus de revêtements convenables qui peuvent résister aux sucs gastriques, c'est-à-dire des solutions de sucre concentrées qui peuvent inclure de la gomme arabique, du talc, une polyvinylpyrrolidone, un polyéthylène glycol et/ou du dioxyde de titane, ou une laque dans des solvants ou mélanges de solvants organiques convenables, ou pour la production de revêtements qui résistent aux sucs gastriques, des solutions et des préparations de cellulose appropriées, comme de phtalate d'acétylcellulose, ou de phtalate d'hydroxypropylméthylcellulose, peuvent être mises en oeuvre. Des colorants, des arômes ou des pigments peuvent être ajoutés aux comprimés ou aux revêtements de dragée, par exemple, dans des buts d'identification ou pour indiquer des doses différentes de composants actifs.

D'autres préparations administrables par voie orale sont des gélules remplies sèches consistant en gélatine et aussi des gélules fermées tendres consistant en gélatine et plastifiants comme le glycérol ou le sorbitol. Les gélules remplies sèches peuvent contenir l'ingrédient actif sous la forme d'un granulat, par exemple, un mélange avec des charges comme le lactose, des liants, comme des amidons et des agents de glissement comme le talc ou le stéarate de magnésium et des stabilisants éventuels. Dans les gélules tendres, l'ingrédient actif est de préférence dissous ou mis en suspension dans des liquides convenables, comme des huiles grasses, des huiles paraffiniques ou des polyéthylène glycols liquides, des stabilisants pouvant êre aussi ajoutés.

Des solutions ou des suspensions aqueuses ou huileuses de l'ingrédient actif, comme un suspension huileuse pour injection, avec un solvant lipophile convenable ou un véhicule comme une huile grasse, par exemple, une huile de sésame ou des esters d'acides gras synthétiques, par exemple, l'oléate d'éthyle, ou des triglycérides, ou des suspensions aqueuses injectables qui contiennent des substances augmentant la viscosité, par exemple une carbone éthylcellulose de calcium, le sorbitol et/ou le dextrane, et éventuellement aussi des stabilisants, conviennent particulièrement pour l'administration parentérale. Les préparations utiles pour la présente invention peuvent être fabriquées par des méthodes classiques, comme par exemple, des procédés de mélange, granulation, fabrication de bonbons, dissolution ou lyophilisation. Par exemple, des préparations pharmaceutiques pour l'administration orale peuvent être obtenues par combinaison de l'ingrédient actif avec des supports solides, granulation éventuelle du mélange résultant et traitement du mélange de granulat, si nécessaire ou désiré, après addition d'adjuvants convenables, pour former des comprimés ou des noyaux de dragée.

Selon un autre mode de réalisation, des combinaisons d'agents systémiques pour lutter contre les endoparasites sont combinés avec les hétérocycles contenant de l'azote de la présente invention pour produire de nouvelles compositions multifonctions pour la lutte contre les parasites. Les agents pour la lutte contre les endoparasites comprennent les avermectines et leurs dérivés, de préférence la milbémycine, l'ivermectine, la milbémycine oxime ou la moxidectine en une dose efficace du point de vue parasiticide. La dose efficace est comprise entre environ 0,01 et environ 100 mg/kg, de préférence entre environ 0,1 et environ 50 mg/kg pour la milbémycine, entre environ 0,01 et environ 10 mg/kg, de préférence entre environ 0,025 et environ 0,5 mg/kg pour l'ivermectine, entre environ 0,01 et environ 100 mg/kg, de préférence entre environ 0,5 et environ 50 mg/kg pour les milbémycine oximes et entre environ 0,01 et environ 100 µg/kg, de préférence entre environ 1,25 et environ 50 µg/kg pour la moxidectine. Des combinaisons particulièrement préférées comprennent une quantité efficace du point de vue ectoparasiticide de pyriproxifen en association avec une quantité efficace du point de vue endectocide d'un composé choisi dans le groupe consistant en milbémycine, dérivés de type milbémycine, ivermectine, dérivés de type ivermectine, milbémycine oxime, dérivés de type milbémycine oxime, moxidectine, dérivés de type moxidectine ou leurs mélanges. Une composition particulièrement préférée comprend une quantité efficace du point de vue ectoparasiticide de pyriproxifen en association avec une quantité efficace du point de vue endectocide de moxidectine et un support acceptable du point de vue pharmaceutique.

Selon un autre mode de réalisation, la présente invention prévoit l'administration à un animal à sang chaud d'une composition comprenant une quantité efficace du point de vue ovicide de pyriproxifen en association avec une quantité efficace du point de vue parasiticide d'un ester macrocyclique choisi dans le groupe consistant en avermectine, milbémycine, milbémycine oxime, ivermectine et moxidectine, de telle sorte que les ingrédients actifs sont transmis aux parasites cibles via le sang de l'animal à sang chaud en des quantités efficaces du point de vue parasiticide suffisantes pour protéger l'animal contre les ectoparasites et les endoparasites.

Un mode de réalisation préféré de la présente invention prévoit l'administration d'une dose efficace du point de vue parasiticide comprenant un mélange d'environ 0,001 à environ 200 mg/kg de pyriproxifen et d'environ 0,5 à environ 100 µg de moxidectine à un animal à sang chaud, de préférence un chien, un chat, une vache, un mouton, une chèvre, un porc, un vison, un lapin, un poulet, un canard ou une oie. Les compositions comprenant les ovicides systémiques hétérocycliques contenant de l'azote et des dérivés d'avermectine de la présente invention, en particulier la composition préférée comprenant la combinaison d'environ 0,2 à environ 100 mg/kg de pyriproxifen et d'environ 1,25 à environ 50 µg de moxidectine, et l'utilisation de la composition comprenant d'environ 0,2 à environ 50 mg/kg de pyriproxifen et d'environ 1,25 à environ 50 µg de moxidectine à un animal à sang chaud, telle que des quantités efficaces du point de vue parasiticide des ingrédients actifs sont transmises aux ectoparasites ou aux endoparasites via le sang de l'animal à sang chaud, sont des modes de réalisation particulièrement préférés de la présente invention.

On s'attend à ce que, lorsque les compositions des combinaisons préférées telles qu'elles sont formulées dans les exemples 11 et 12 ci-après, sont administrées à des chiens infestés de puces qui sont maintenus dans un environnement favorable au développement des larves, à leur nymphose et à l'émergence de nouvelles puces pour réinfester les chiens, que les chiens sont aussi exposés en continu à une infection par le filaire cardio-pulmonaire du chien par la morsure des moustiques transmettant les larves infectieuses de *D. immitis* et que les chiens sont observés pendant 6 mois, les animaux d'essai soient pratiquement exempts de puces et de filaire cardio-pulmonaire à la fin de la période de test, tandis que des chiens témoins traités de façon similaire souffrent de graves infestations croissantes de puces et de filaire cardio-pulmonaire du chien. On pense aussi que les compositions ecto- et endoparasiticides vont offrir une large protection contre les puces, les tiques, les moustiques, les poux, les acariens de la gale et les mouches qui piquent, ainsi que des effets antihelminthiques et antiparasitiques contre les parasites métazoaires connus en tant qu'Helminthes, en particulier Nematoda et des membres de la famille *Filaridae* ou *Setariidae,* et les genres *Dirofilaria* chez les chiens, et les *Nematospiroides, Syphacia* et *Aspiculuris* chez les rhodentias. On prévoit que des combinaisons de pyriproxifen et de milbémycine, milbémycine oxime, ivermectine ou moxidectine, peuvent aussi présenter des effets accrûs ou synergiques, selon lesquels un ou plusieurs des composants des mélanges sont plus efficaces que la même dose du produit administré seul. Les exemples suivants illustrent l'invention, mais sans la limiter.

### Exemple 1 : Comprimés (1000)

Des comprimés contenant 25 mg d'ingrédient actif, par exemple la 2-[1-méthyl-2-(4-phénoxyphénoxy)-éthoxy]-pyridine (pyriproxifen), peuvent être préparés de la façon suivante :

| Constituants | Quantité (g) |
|---|---|
| Ingrédient actif | 25 |
| Lactose | 100,7 |
| Amidon de blé | 7,5 |
| Polyéthylène glycol (PM 6000) | 5,0 |
| Talc | 5,0 |
| Stéarate de magnésium | 1,8 |
| Eau déminéralisée | q.s. |

Tous les composants solides sont d'abord forcés à travers un tamis ayant une ouverture de maille de 0,6 mm, puis l'ingrédient actif, le lactose, le talc, le stéarate de magnésium et la moitié de l'amidon, sont mélangés. L'autre moitié de l'amidon est mise en suspension dans 40 ml d'eau et la suspension est ajoutée à une solution à ébullition du polyéthylène glycol dans 100 ml d'eau. La base d'amidon résultante est ajouté au lot principal et le mélange, si nécessaire avec addition d'eau, est granulé. Le granulat est séché pendant une nuit à 35°C, forcé à travers un tamis ayant une ouverture de maille de 1,2 mm et pressé en comprimés.

### Exemple 2 : Comprimés

Des comprimés contenant 20 mg d'ingrédient actif, par exemple la 2-[1-méthyl-2-(4-phénoxyphénoxy)-éthoxy]-pyridine (pyriproxifen), peuvent être préparés de la façon suivante :

| Constituants | Quantité (g) |
|---|---|
| Ingrédient actif | 200,0 |
| Lactose | 290,8 |
| Amidon de pomme de terre | 274,7 |
| Acide stéarique | 10,0 |
| Talc | 200,0 |
| Stéarate de magnésium | 2,5 |
| Silice colloïdale | 30,0 |
| Ethanol | q.s. |

Le mélange d'ingrédient actif, de lactose et de 194,70 g d'amidon de pomme de terre est humidifié avec une solution éthanolique de l'acide stéarique et granulé à travers un tamis. Après séchage, le restant de l'amidon de pomme de terre, le talc, le stéarate de magnésium et la silice colloïdale sont ajoutés, mélangés aux mélanges et pressés en comprimés de 0,1 g qui, si cela est désiré, peuvent être pourvus de rainures de rupture pour un meilleur ajustement du dosage.

### Exemple 3 : Gélules

Des gélules contenant 25 mg d'ingrédient actif, par exemple la 2-[1-méthyl-2-(4-phénoxyphénoxy)-éthoxy]-pyridine (pyriproxifen), peuvent être préparées de la façon suivante :

| Constituants | Quantité (g) |
|---|---|
| Ingrédient actif | 25,0 |
| Lactose | 249,0 |
| Gélatine | 2,0 |
| Amidon de maïs | 10,0 |
| Talc | 15,0 |
| Eau | q.s. |

L'ingrédient actif est mélangé au lactose et le mélange est humidifié uniformément avec une solution aqueuse de la gélatine et granulé à travers un tamis ayant une ouverture de maille de 1,2-1,5 mm. Le granulat est ensuite mélangé à l'amidon de maïs séché et le talc, puis introduit en portions de 300 mg dans des gélules de gélatine dure (taille 1).

### Exemple 4 : Pré-mélange (additif alimentaire)

0,25 partie en poids d'ingrédient actif, par exemple de pyriproxifen, et
4,75 parties de phosphate de calcium secondaire ou de kaolin, aérosil ou carbonate ou chaux, mélangées de façon homogène avec
95 parties d'aliment pour animaux.

### Exemple 5 : Concentrat émulsifiable

20 parties en poids d'ingrédient actif, par exemple de pyriproxifen, sont mélangées avec :
20 parties d'émulsifiant (par exemple, un mélange d'alkylarylpolyglycol éther et d'alkylarylsulfonates) et
60 parties de solvant jusqu'à obtention d'une solution homogène. En diluant ce concentrat avec de l'eau, il est possible d'obtenir une émulsion ayant la concentration désirée.

### Exemple 6 : Poudre soluble

20 parties en poids d'ingrédient actif, par exemple de pyriproxifen;
1 partie de lauryl sulfate de sodium;
3 parties de silice colloïdale; et
71 parties d'urée.

Les constituants sont mélangés et le mélange est finalement broyé dans un broyeur convenable.

### Exemple 7 : Gels :

| Constituants | Quantité (g) |
|---|---|
| Ingrédient actif | 0,5 |
| Huile de foie de morue | 12,5 |
| Huile de maïs | 124,5 |
| Peptone de boeuf | 2,5 |
| Mélasses | 5,0 |
| Glucose | 12,5 |
| Benzoate de sodium | 5,0 |
| Méthyl cellulose | 12,5 |
| Eau déminéralisée | 75,0 |

L'ingrédient actif, par exemple le pyriproxifen, est dissous dans la phase d'huile. Le glucose, la peptone, le benzoate de sodium et les mélasses sont dissous dans l'eau déminéralisée chauffée à 60°C dans laquelle la méthyl cellulose est mélangée pour former un gel, qui est ensuite mélangé à la phase huileuse. Le gel est introduit dans des tubes à presser en matière plastique flexible qui sont fermés par sertissage et bouchés. Dans une variante, le gel peut être introduit dans des seringues de type "composer une dose" graduées pour fournir le taux d'administration quotidien désiré par kg de poids corporel, par exemple 1 g/10 kg de poids corporel. Ce gel de base sous forme de dose orale quotidienne peut être amélioré par addition de vitamines (par exemple, A, D, E et le groupe B), de sels minéraux, d'oligoéléments et d'acides gras essentiels, pour fournir un supplément nutritionnel complet facile à administrer aux animaux familiers, qui permet aussi de lutter efficacement contre les ectoparasites. Les proportions de composants indiquées plus haut fournissent 2,0 mg d'ingrédient actif par gramme de gel.

### Exemple 8 : Supplément alimentaire:

| Constituants | Quantité (g) |
|---|---|
| Ingrédient actif | 1,0 |
| Huile de poisson (Omega 3) | 80,0 |
| Huile de bourrache (acide γ-linoléique) | 20,0 |
| Huile de tournesol | 847,9 |
| Arôme de poulet | 5,0 |
| Hydroxytoluène butylé | 1,0 |
| Propylène glycol | 20,0 |
| Sulfate de zinc | 1,0 |
| Acétate de tocophérol | 3,0 |
| Inositol | 0,5 |
| Chlorhydrate de pyridoxine | 0,1 |
| Palmitate de vitamine A | 0,1 |
| Biotine | 0,1 |
| Parabenzoates de propyle-méthyle | 0,3 |

Tous les ingrédients sont dissous/mélangés dans la phase huileuse. Le supplément liquide est introduit dans des bouteilles d'un litre du type "bascule et mesure" à presser en matière plastique souple, qui sont bouchées. Le dosage quotidien administré sous forme d'additif alimentaire liquide pour chiens et chats, dépend du poids corporel et du taux d'administration quotidien désiré (mg d'ingrédient actif par kg de poids corporel, ou oz/25 lb de poids corporel). L'administration de suppléments actifs du point de vue dermatologique, de type acides gras essentiels/vitamine/zinc, est particulièrement bénéfique pour les animaux familiers souffrant de dermite provoquée par l'exposition à des infestations d'ectoparasites. Les proportions des composés indiquées plus haut fournissent 1 mg d'ingrédient actif, par exemple, le pyriproxifen, par gramme de supplément alimentaire.

### Exemple 9 : Implant monolithe de 150 mg :

| Constituants | Quantité (g) |
|---|---|
| Ingrédient actif | 30,0 |
| Polymère hydrophile méthacrylique | 30,0 |
| Composition silicone élastomère | 90,0 |

L'ingrédient actif, par exemple le pyriproxifen, est mélangé avec la poudre de copolymère hydrophile préparée à laquelle sont ajoutés le composite silicone, par exemple, un hydroxy-hydrogène-poly(diméthylsiloxane) et un agent de réticulation, le méthyltriacétoxysiloxane, selon les proportions indiquées. Le mélange est laissé réagir à la température ambiante dans un moule pendant 12 heures. Les implants sont stérilisés par des rayons gamma ou par gazage avec de l'oxyde d'éthylène après conditionnement. Les implants sont insérés de façon sous-cutanée soit par un trocart, soit par une procédure chirurgicale mineure. Plusieurs implants peuvent être insérés simultanément en fonction du poids de l'animal, du taux de libération connu de l'ingrédient actif et de la fréquence de réimplantation désirée. L'unique implant de l'exemple, fournit des concentrations adéquates dans le sang pendant 90 jours pour stériliser tous les oeufs pondus par des puces femelles se nourrissant sur un chat de 3 kg ayant un implant.

### Exemple 10 : Gel transdermique

| Constituants | Quantité (g) |
|---|---|
| Ingrédient actif | 12,5 |
| Huile de maïs | 12,5 |
| Diméthylsulfoxyde | 12,5 |
| Hydroxytoluène butylé | 1,3 |
| Méthyl cellulose | 5,0 |
| Eau déminéralisée | 206,3 |

L'ingrédient actif est dissous dans la phase huileuse à laquelle sont ajoutés les autres excipients et le mélange est mélangé en un gel lisse ayant une viscosité de 1000-1200 centipoises. Le gel est introduit en aliquots de 2 ml dans des tubes à presser de matière plastique souple fournissant une dose unitaire, qui sont fermés à chaud. Dans une variante, le gel peut être introduit dans des seringues multi-doses du type "composer une dose" graduées et ajustables pour fournir, au taux d'administration approprié, la dose mensuelle désirée, pour l'application topique directe sur la peau du dos de l'animal. Les proportions des composés indiquées plus haut fournissent 50 mg d'ingrédient actif par gramme de gel transdermique.

D'autres agents ou composants ayant une activité biocide, qui sont inertes vis-à-vis des ingrédients actifs et acceptables pour les animaux à traiter, ou des sels minéraux ou des vitamines, peuvent être mélangés aux compositions décrites plus haut. D'une façon analogue à celle qui est décrite dans les exemples 1 à 10, il est possible de fabriquer des préparations correspondantes contenant un quelconque composé choisi dans le groupe de composés hétérocycliques contenant de l'azote tels que définis plus haut. Les composés préférés sont ceux dans lesquels R₁ est une pyridine substituée en position 2, une pyrimidine ou un 1,3-triazole; des composés particulièrement préférés sont les pyridines à substituant 2-(4-phénoxyphénoxy)-éthoxy et le composé le plus avantageux est la 2-[1-méthyl-2-(4-phénoxyphénoxy)-éthoxy]-pyridine. Dans une variante, les composés hétérocycliques contenant de l'azote peuvent être combinés avec un antibiotique comme l'avermectine, comme cela est décrit ci-après.

### Exemple 11 : Lutte contre les puces par administration orale d'un ingrédient actif

Du pyriproxifen de qualité technique, la 2-[1-méthyl-2-(4-phénoxyphénoxy)-éthoxy]-pyridine, est dilué dans de l'huile de maïs et introduit dans des gélules de gélatine. Les charges d'ingrédient actif sont calculées pour fournir des doses nominales de 0,2, 2,0 et 20 mg par kg de poids corporel pour chacun de 6 chats, groupés en paires. Les sujets du test sont de bons hôtes pour les puces, dociles et acceptant la prise de pilule orale quotidienne. Les chats sont placés dans des cages individuelles avec des collecteurs d'oeufs de puce, de l'eau et de la nourriture à volonté, deux autres chats sont traités de la même façon en tant que témoins. La production d'oeufs est surveillée par 2 collections faites avant le traitement des animaux. Des doses d'ingrédients actifs sont données les jours 5, 11-15 et 18-22 d'une période de test de 28 jours. Les oeufs de puce sont collectés et cultivés sur un milieu d'élevage convenable pour les larves. Le taux d'éclosion et les taux d'émergence des puces adultes sont mesurés. L'ingrédient actif est administré sous forme d'une gélule d'huile de maïs. Le nombre de nymphes et de puces adultes ayant éclos est déterminé (Tableaux 1 et 2).

Les données montrent une stérilisation partielle ou complète des oeufs de puce à toutes les doses, comme cela est mesuré par les nombres d'éclosion et de larves ou par le développement complet et l'émergence d'adultes. Les effets stérilisants, qu'ils soient mesurés par les comptes d'oeufs éclos-larves ou par l'émergence des puces adultes, sont essentiellement similaires, sauf que la fertilité apparente des oeufs collectés avant la première dose et à partir des nombres déterminés chez les témoins partout, montre une fertilité supérieure basée sur les comptes de larves par rapport à la fertilité basée sur l'émergence des puces adultes. Une stérilité complète des oeufs de puce à partir du jour 14 à tous les taux de dosage est observée. Aux doses de 2 mg/kg et 20 mg/kg, une stérilisation complète des oeufs est notée le jour 12. Le cycle de vie de la puce est ainsi brisé et une lutte contre les puces est réalisée.

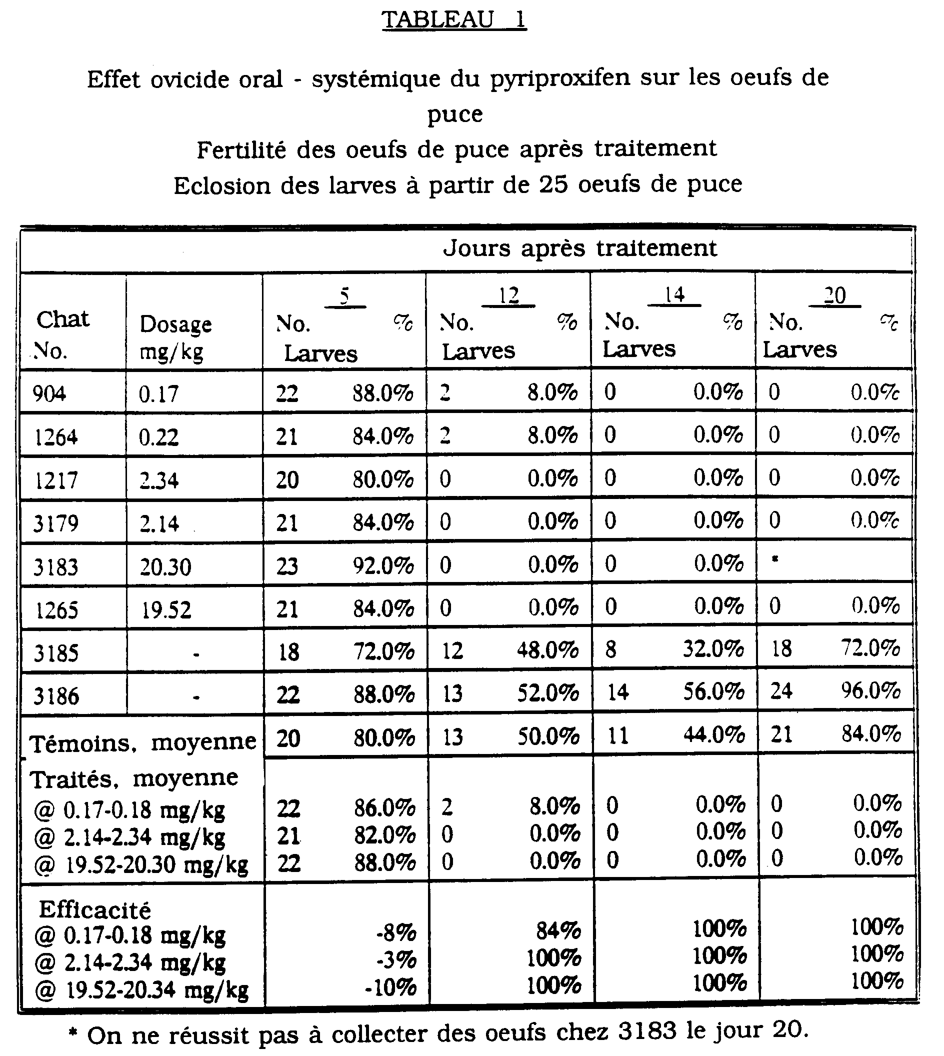

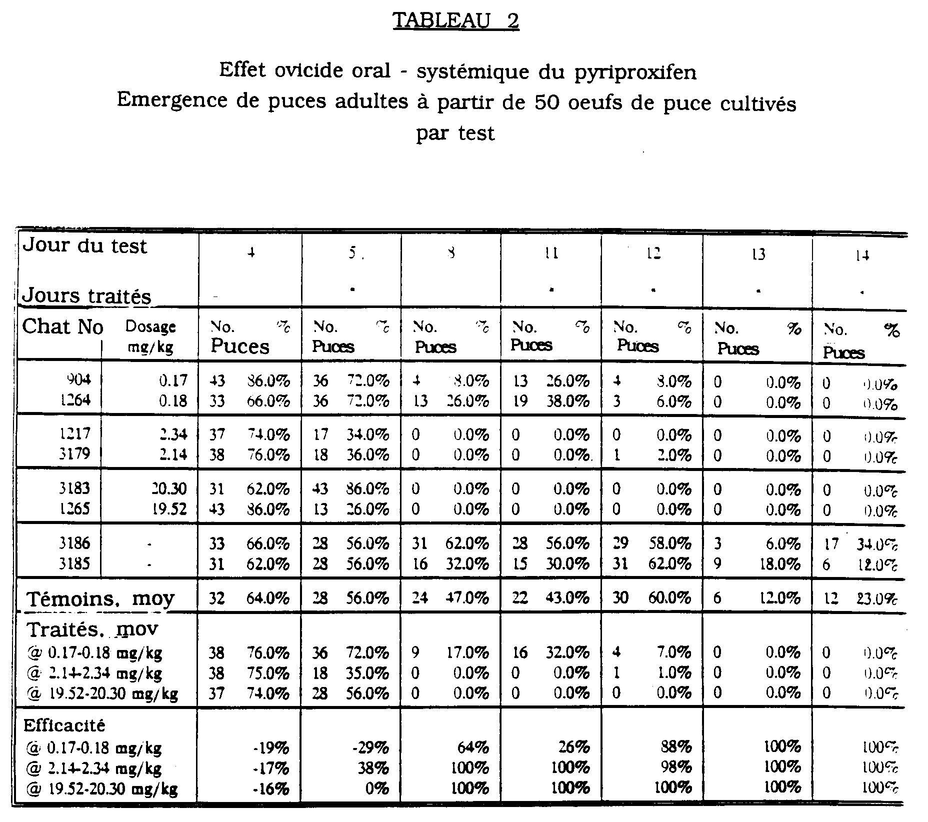

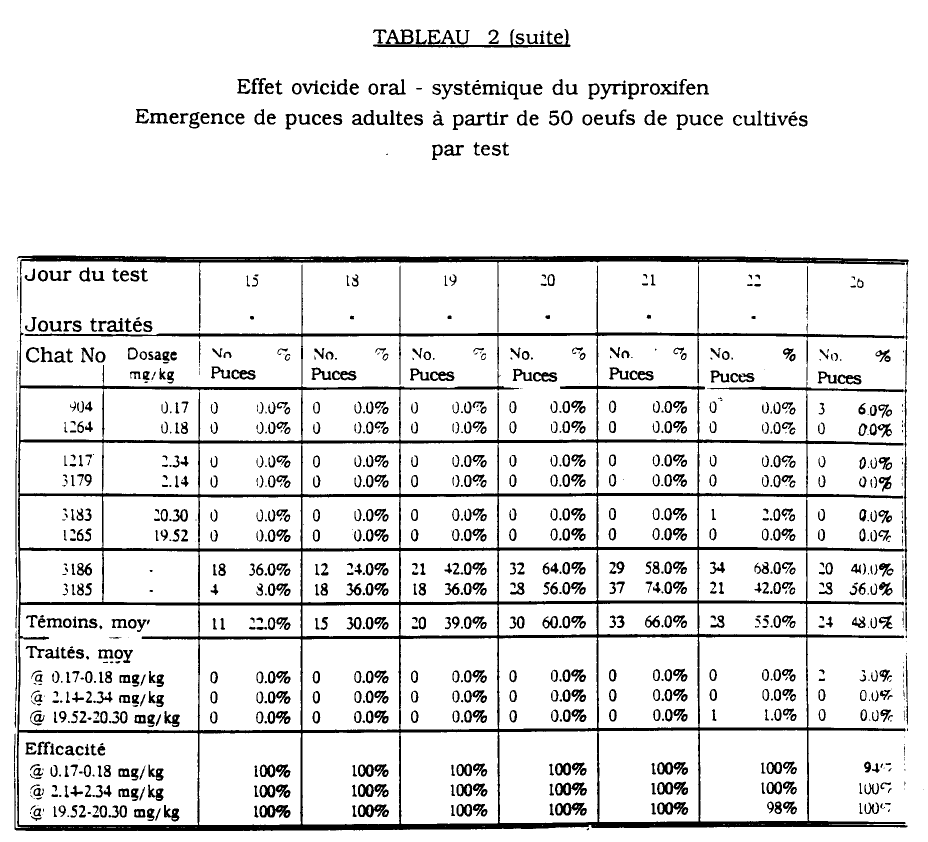

La lutte efficace contre *Acarina* est mise en évidence dans les brevets US 4.920.222, 4.879.292 et 4.251.223 avec les araignées rouges femelles. Les données de la présente invention indiquent que de façon surprenante, les composés de la présente invention fournissent un contrôle efficace systémique contre les ectoparasites *Acarina* via le sang des animaux hôtes traités.

Des résultats comparables à ceux de l'exemple 11 sont obtenus lorsque les formulations des exemples 1 à 10 sont administrées à des animaux d'essai avec les autres composés décrits ci-dessus, en tant qu'ingrédients actifs. Une activité ovicide systémique contre d'autres ectoparasites présentée par les ingrédients actifs identifiés plus haut, est observée lorsque le composé de test est administré selon la procédure de l'exemple 9 et que les ectoparasites sont laissés se nourrir sur l'animal d'essai, même lorsque l'animal d'essai est un hôte pour une espèce particulière.

### Exemple 12 : Lutte contre les puces par administration d'une seule dose orale d'ingrédient actif.

Le pyriproxifen est administré à 6 chats essentiellement selon la procédure de l'exemple 11, sauf qu'une dose unique de pyriproxifen (environ 2,0, 20,0 ou 50,0 mg/kg) ou de placebo (huile de maïs dans des gélules de gélatine) est administrée à la place du traitement par plusieurs doses. Le nombre de nymphes et de puces adultes ayant éclos est déterminé (Tableaux 3 et 4). Les résultats montrent qu'il y a une relation entre la dose et la réponse dans l'éclosion des oeufs après traitement. La réapparition d'oeufs fertiles est la plus précoce pour la dose la plus faible (2 mg/kg, le jour 9), tandis que le plus grand intervalle de temps avant réapparition d'oeufs fertiles est noté avec la plus forte dose (50 mg/kg, le jour 25).

L'émergence des puces adultes est analogue (Tableau 4). Une dose unique de 2 mg/kg empêche l'émergence pendant 7 jours,, une dose de 20 mg/kg est efficace jusqu'aux jours 17 ou 29 et une dose de 50 mg/kg jusqu'aux jours 25 ou 28 après traitement.

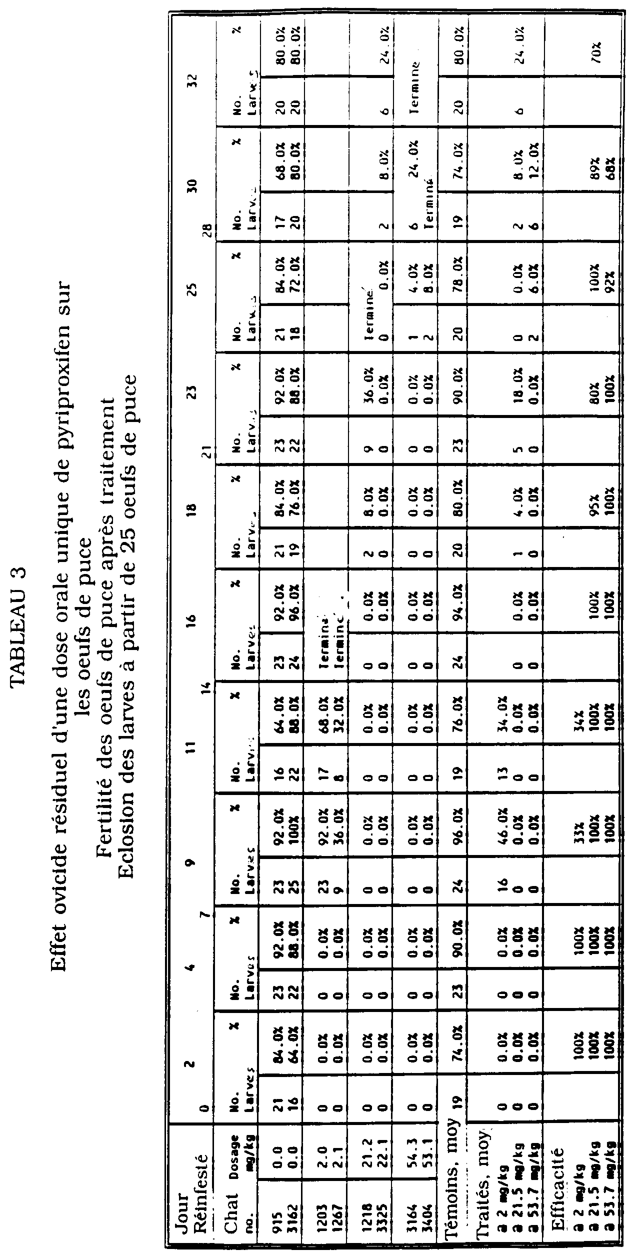

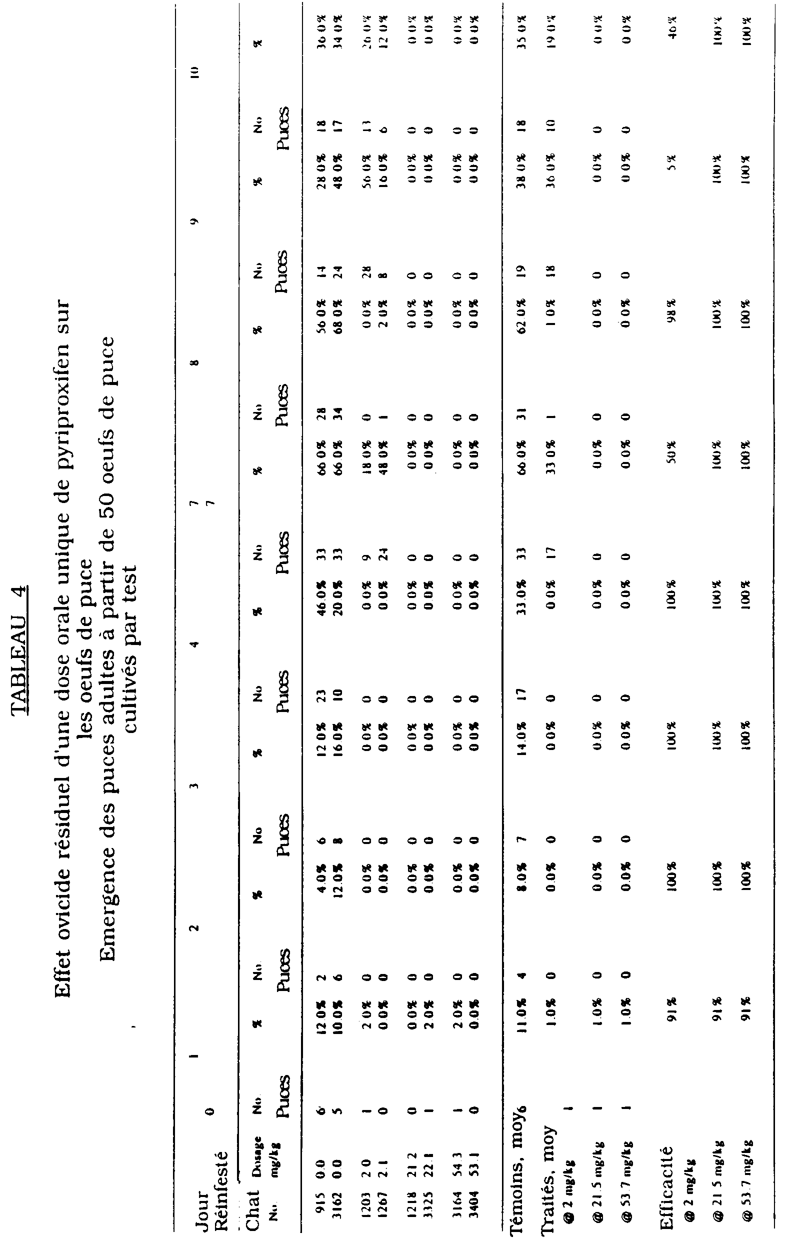

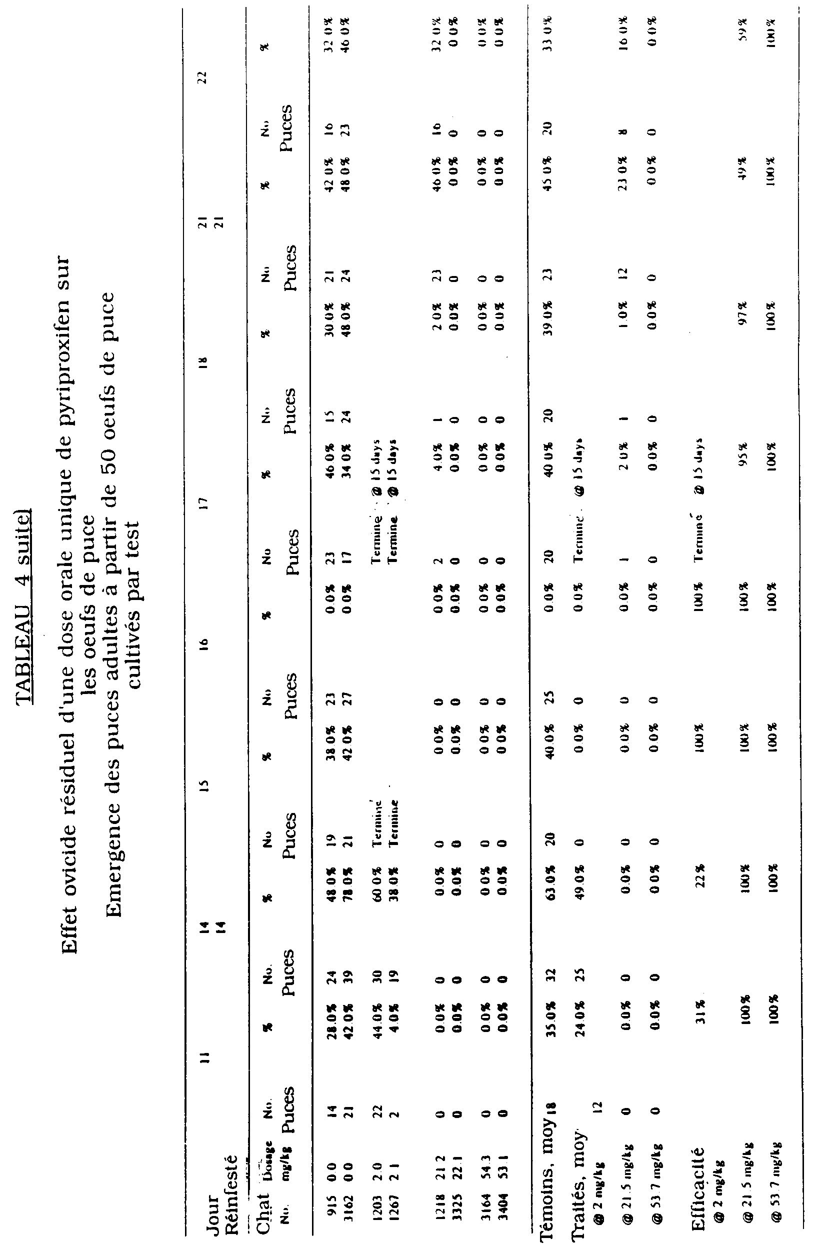

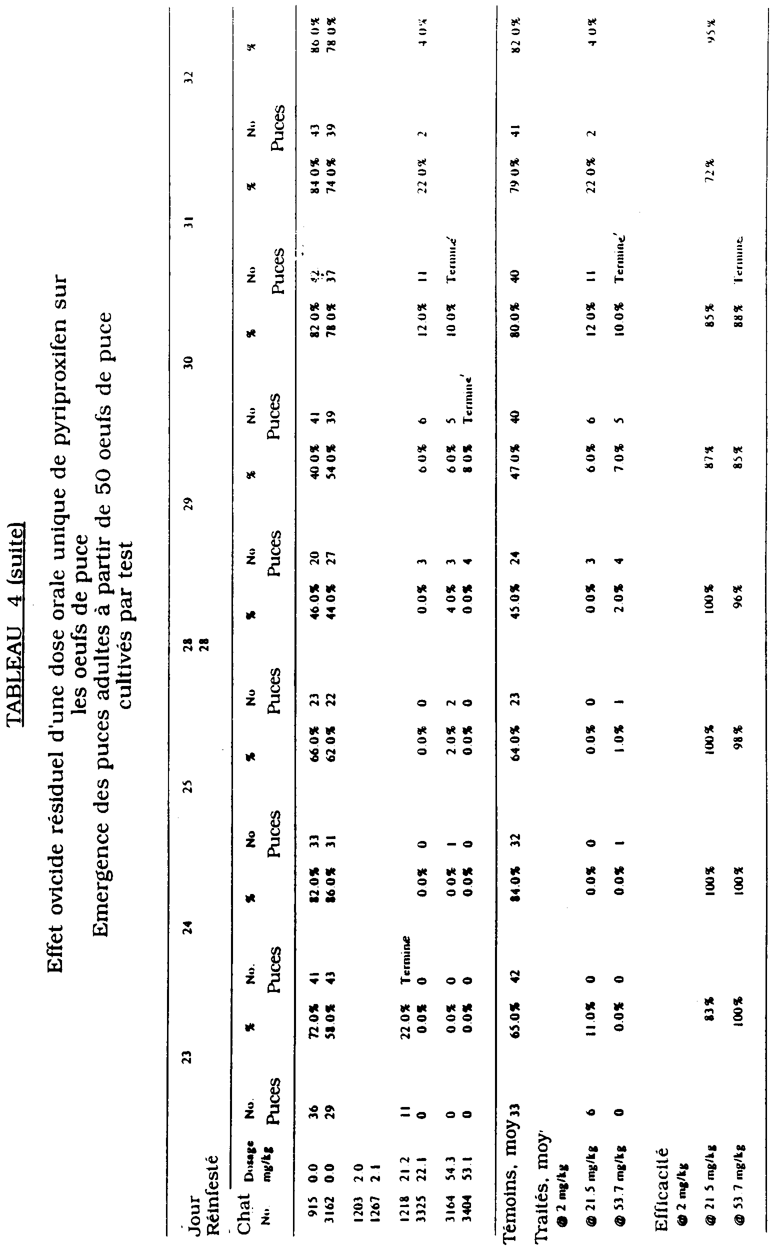

### Exemple 13 : Lutte contre les tiques par administration orale d'un ingrédient actif

Du pyriproxifen de qualité technique, la 2-[1-méthyl-2-(4-phénoxyphénoxy)-éthoxy]-pyridine, est dilué dans de l'huile de maïs et introduit dans des gélules de gélatine à deux concentration, 5% et 25%. Les charges d'ingrédient actif sont calculées pour fournir des doses nominales d'environ 30 et environ 175 mg par kg de poids corporel pour chacun de 4 lapins, à raison de 2 lapins par groupe pour chaque concentration. Les sujets du test sont de bons hôtes pour les tiques. Les solutions de pyriproxifen dans l'huile de maïs sont administrées par gavage à 4 lapins, à raison de 2 par groupe pour chaque concentration (Tableau 5). Deux autres lapins reçoivent de façon analogue de l'huile de maïs comme placebo.

Dix tiques (*Dermacentor variabilis*) sont appliquées sur chaque oreille de chaque lapin et enfermées dans des sacs en coton pour oreille pour retenir les tiques et permettre leur récupération lorsqu'elles sont gonflées de sang. Après application des tiques, les sacs pour oreille sont examinés chaque jour et les moments auxquels les tiques mâles et femelles sont gonflées de sang et se détachent, sont notés (Tableau 6).

Six tiques femelles gonflées de sang, collectées de façon aléatoire à partir de chaque groupe (A, B et C, 18 au total) sont confinées. Lorsque les oeufs sont pondus, 100 oeufs de chaque tique sont observés tous les 5 jours jusqu'à 10 jours après obtention des nombres maxima d'éclosion de larves (Tableau 7).

**TABLEAU 5**

| Effet régulateur de la croissance des insectes du pyriproxifen par voie orale sur la fertilité des oeufs de tique Traitements des lapins et dosages | | | | | | | |
|---|---|---|---|---|---|---|---|
| Lapin No | Poids lapin (kg) | Groupe/solution Code | Sering. poids (g) av/ap. | Dose de solution | | Dose Pyriprox. | |
| | | | | g | g/kg | total mg | taux mg/kg |
| 1 | 2.75 | A/22.3s1 | 7.818 | 1.851 | 0.673 | 92.57 | 33.66 |
| | | | 5.967 | | | | |
| 2 | 2.90 | A/22.3s1 | 7.816 | 1.867 | 0.644 | 93.37 | 32.20 |
| | | | 5.949 | | | | |
| 3 | 2.75 | B/22.3s2 | 7.935 | 1.983 | 0.721 | 498.4 | 181.25 |
| | | | 5.952 | | | | |
| 4 | 2.95 | B/22.3s2 | 7.887 | 2.003 | 0.679 | 503.5 | 170.66 |
| | | | 5.884 | | | | |
| 5 | 2.55 | C/22.3s3 | 8.566 | 1.838 | 0.721 | 0 | 0.00 |
| | | | 6.728 | | | | |
| 6 | 3.00 | C/22.3s3 | 7.764 | 1.866 | 0.622 | 0 | 0.00 |
| | | | 5.898 | | | | |

**TABLEAU 6**

| Effet régulateur de la croissance des insectes du pyriproxifen par voie orale sur la fertilité des oeufs de tique Fixation/gonflement de sang des tiques | | | | | | | | | | | | | |
|---|---|---|---|---|---|---|---|---|---|---|---|---|---|
| Lapin No. | No. tiques fixées | | | | gonflées/détachées cumulatives | | | | | | | | |
| | Or. gauche | | Or. droite | | Jours après fixation | | | | | | | | |
| | Mâle | Fem. | Mâle | Fem. | 3 | 4 | 5 | 6 | 7 | 8 | 9 | 10 | 11 |
| 1 | 9 | 9 | 7 | 1 | 0 | 5 | 6 | 8 | 10 | 10 | 11 | 12 | 12 |
| 2 | 9 | 2 | 5 | 0 | 0 | 0 | 1 | 1 | 1 | 1 | 1 | 1 | 1 |
| 3 | 9 | 8 | 6 | 5 | 3 | 10 | 11 | 12 | 14 | 14 | 14 | 14 | 14 |
| 4 | 10 | 10 | 4 | 5 | 1 | 11 | 11 | 11 | 12 | 12 | 12 | 12 | 12 |
| 5 | 9 | 8 | 10 | 9 | 0 | 4 | 7 | 7 | 10 | 11 | 12 | 13 | 14 |
| 6 | 10 | 10 | 10 | 9 | 0 | 5 | 13 | 13 | 14 | 14 | 14 | 14 | 14 |

**TABLEAU 7**

| Effet régulateur de la croissance des insectes du pyriproxifen par voie orale sur la fertilité des oeufs de tique Eclosion d'oeufs de tique, à partir de 6 tiques femelles par groupe de lapins | | | | | | | | | | | |
|---|---|---|---|---|---|---|---|---|---|---|---|
| Groupe | Tique | Eclosion cumulative d'oeufs de tique (%) les jours après la date du premier gonflement de sang | | | | | | | | | |
| | | 29 | 33 | 38 | 43 | 48 | 53 | 58 | 63 | Moy. groupe ± E. T. | Efficacité traitement |
| A | 1 | 0 | 30 | 89 | 91 | 91 | 91 | 91 | 91 | 81.50 +/-11.70 | 17.8% |
| | 2 | 0 | 1 | 63 | 71 | 71 | 71 | 71 | 71 | | |
| | 3 | 0 | 48 | 67 | 67 | 67 | 67 | 67 | 67 | | |
| | 4 | 0 | 1 | 91 | 92 | 92 | 92 | 92 | 92 | | |
| | 5 | 0 | 0 | 72 | 72 | 72 | 72 | 72 | 72 | | |
| | 6 | 0 | 5 | 96 | 96 | 96 | 96 | 96 | 96 | | |
| B | 1 | 0 | 3 | 85 | 86 | 86 | 86 | 86 | 86 | 47.17 +/-27.39 | 52.4% |
| | 2 | 0 | 22 | 63 | 47 | 67 | 67 | 67 | 67 | | |
| | 3 | 0 | 7 | 24 | 25 | 25 | 26 | 26 | 26 | | |
| | 4 | 0 | 0 | 4 | 4 | 4 | 5 | 5 | 5 | | |
| | 5 | 0 | 41 | 63 | 63 | 63 | 63 | 63 | 63 | | |
| | 6 | 0 | 1 | 36 | 36 | 16 | 36 | 36 | 36 | | |
| C | 1 | 0 | 0 | 94 | 97 | 97 | 97 | 97 | 97 | 99.17 +/-1.07 | 0.0% |
| | 2 | 0 | 6 | 100 | 100 | 100 | 100 | 100 | 100 | | |
| | 3 | 0 | 0 | 100 | 100 | 100 | 100 | 100 | 100 | | |
| | 4 | 0 | 20 | 99 | 99 | 99 | 99 | 99 | 99 | | |
| | 5 | 0 | 0 | 94 | 99 | 99 | 99 | 99 | 99 | | |
| | 6 | 0 | 0 | 90 | 97 | 100 | 100 | 100 | 100 | | |

Les tiques s'attachent et se gonflent facilement de sang sauf sur le lapin No 2, sur lequel une seule tique gonflée de sang est récupérée. Les données d'éclosion d'oeufs (Tableau 7) montrent que 40 jours après le gorgement de sang, la plupart des oeufs de tique ont éclos. L'efficacité est observée au taux de dosage supérieur, puisque la fertilité des oeufs est réduite à environ la moitié par rapport à la fertilité des oeufs provenant de tiques qui sont nourris sur des lapins traités avec le placebo. Une réduction marginale de la fertilité des oeufs est aussi observée chez les oeufs pondus par des tiques qui sont nourris sur des lapins traités au taux de dosage inférieur. Cependant, en raison de la forte variation de la fertilité des oeufs dans les groupes, allant d'une stérilité presque complète (5%) à une réduction marginale (86%, par rapport au contrôle correspondant à 100%), les différences entre les fertilités des oeufs dans les groupes ne sont pas statistiquement significatives au niveau de probabilité de 95%, aux doses testées. Néanmoins, dans le cas du traitement à la dose supérieure, 1 des 6 tiques est virtuellement stérilisée (4-5% des oeufs fertiles), 2 de plus sont partiellement stérilisées (24-36% des oeufs fertiles) et les 3 restantes sont partiellement stérilisées (63-86% des oeufs fertiles), tandis que la fertilité des oeufs témoins en même temps dépasse 94%, et surtout 99-100% pour les tiques nourries sur les lapins témoins. Ces données suggèrent qu'une stérilisation complète des tiques peut être attendue à des doses supérieures à celles qui sont testées dans cette expérience.

### Exemple 14 : Gel en combinaison

| Composition | Quantité (g) |
|---|---|
| Pyriproxifen | 50,0 |
| Milbémycine oxime | 1,3 |
| Huile de foie de morue | 12,5 |
| Huile de maïs | 73,8 |
| Peptone de boeuf | 2,5 |
| Mélasses | 5,0 |
| Glucose | 12,5 |
| Benzoate de sodium | 5,0 |
| Méthyl cellulose | 12,5 |
| Eau déminéralisée | 75,0 |

Le pyriproxifen et la milbémycine oxime sont dissous dans la phase d'huile. Le glucose, la peptone, le benzoate de sodium et les mélasses sont dissous dans l'eau déminéralisée chauffée à 60°C dans laquelle la méthyl cellulose est mélangée pour former un gel, qui est ensuite mélangé à la phase huileuse. Le gel est introduit dans des tubes à presser en matière plastique flexible qui sont fermés par sertissage et bouchés. Dans une variante, le gel peut être introduit dans des seringues de type "composer une dose" graduées pour fournir le taux de dose quotidien désiré par kg de poids corporel.

Ce gel de base sous forme de dose orale quotidienne peut être amélioré par addition de vitamines (par exemple, A, D, E et le groupe B), de sels minéraux, d'oligoéléments et d'acides gras essentiels, pour fournir un supplément nutritionnel complet facile à administrer à des animaux familiers, qui fournit aussi une prophylaxie efficace contre le filaire cardio-pulmonaire du chien, les ankylostomes et les ascarides et permet de lutter contre les ectoparasites, par administration mensuelle d'un gramme par 10 kg de poids corporel.

### Exemple 15 : Implant monolithe de 300 mg :

| Composition | Quantité (g) |
|---|---|
| Pyriproxifen | 30,0 |
| Moxidectine | 1,0 |
| Polymère hydrophile méthacrylique | 60,0 |
| Composite silicone élastomère | 240,0 |

L'ingrédient actif est mélangé avec la poudre de copolymère hydrophile préparée à laquelle sont ajoutés le composite silicone, par exemple, un hydroxy-hydrogènepoly(diméthylsiloxane) et un agent de réticulation, le méthyltriacétoxysiloxane, selon les proportions indiquées. Le mélange est laissé réagir à la température ambiante dans un moule pendant 12 heures. Les implants sont stérilisés par des rayons gamma ou par gazage avec de l'oxyde d'éthylène après conditionnement. Les implants sont insérés de façon sous-cutanée soit par un trocart, soit par une procédure chirurgicale mineure. Plusieurs implants peuvent être insérés simultanément en fonction du poids de l'animal, du taux de libération connu d'ingrédient actif et de la fréquence de réimplantation désirée. L'unique implant de l'exemple fournit des taux adéquats dans le sang pendant 90 jours pour stériliser tous les oeufs pondus par les puces femelles et prévenir la transmission du filaire cardio-pulmonaire par tous les moustiques se nourrissant sur un chat de 6 kg ayant un tel implant. Des implants multiples ou plus gros peuvent être administrés à des chiens plus gros, et les implants peuvent être facilement enlevés après libération totale du composé. En modifiant les caractéristiques physiques des implants, on peut obtenir des taux de libération différents et/ou des durées de libération plus longues.

## Revendications

1. Utilisation d'un composé choisi dans le groupe ayant la formule générale : 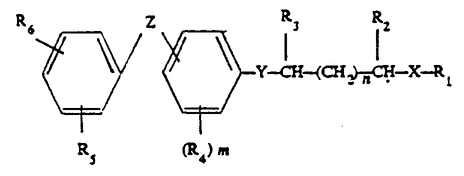 dans laquelle R₁ est l'un des groupes suivants : 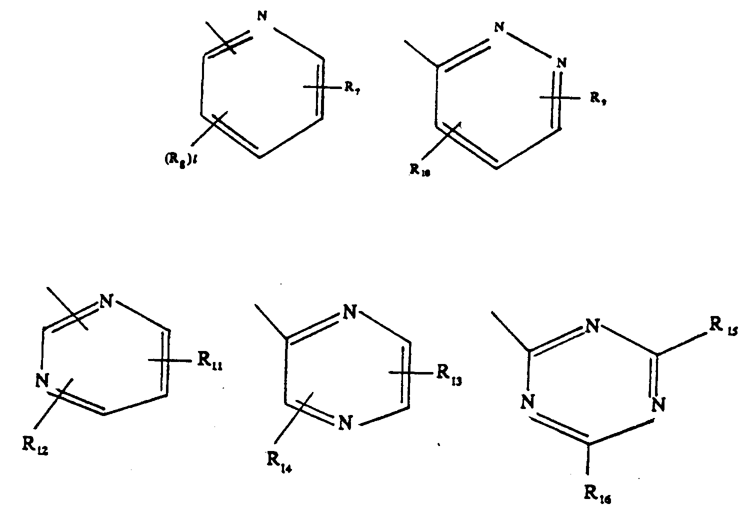 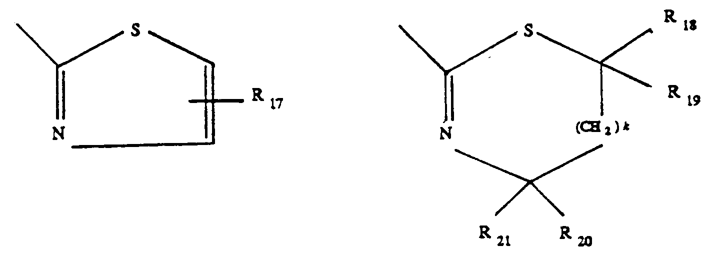 dans lesquels R₇, R₈, R₉, R₁₀, R₁₁, R₁₂, R₁₃, R₁₄, R₁₅, R₁₆ et R₁₇, identiques ou différents, sont chacun un atome d'hydrogène ou d'halogène, un groupe alcoxy en C₁₋₄, alkylthio en C₁-₄, trifluorométhyle ou nitro ; R₁₈, R₁₉, R₂₀ et R₂₁, identiques ou différents, sont chacun un atome d'hydrogène ou un groupe méthyle, *k* est un entier de 0 à 1 et *l* est un entier de 0 à 3 ; R₂ et R₃, identiques ou différents, sont chacun un atome d'hydrogène ou d'halogène ou un groupe méthyle ; R₄ est un atome d'halogène ou un groupe méthyle ; R₅ et R₆, identiques ou différents, sont chacun un atome d'hydrogène ou d'halogène, un groupe haloalkyle en C₁₋₄ , ou haloalcoxy en C₁₋₄ ; X, Y et Z, identiques ou différents, sont chacun un atome d'oxygène, de soufre ou un groupe méthylène, *m* est un entier de 0 à 4, et *n* est un entier de 0 à 2, pour la préparation d'une composition pharmaceutique destinée à la lutte systémique des ectoparasites qui attaquent les animaux à sang chaud.

2. Utilisation selon la revendication 1, dans laquelle le composé utilisé a pour R₁ une pyridine substituée en position 2.

3. Utilisation selon la revendication 2, dans laquelle le composé utilisé est une (4-phénoxyphénoxy)-éthoxy pyridine.

4. Utilisation selon la revendication 1, dans laquelle le composé utilisé est la 2-[1-méthyl-2-(4-phénoxyphénoxy)-éthoxy] pyridine.

5. Utilisation selon la revendication 1, dans laquelle la composition est adaptée a l'administration d'une dose de composé comprise entre environ 0,0001 mg et environ 1000 mg par kg de poids corporel de l'animal à sang chaud.

6. Utilisation selon la revendication 1, dans laquelle la composition est adaptée à l'administration d'une dose de composé comprise entre environ 0,01 mg et environ 100 mg par kg de poids corporel de l'animal a sang chaud.

7. Utilisation selon la revendication 1, dans laquelle la composition est adaptée a l'administration d'une dose de composé comprise entre environ 0,02 mg et environ 50 mg par kg de poids corporel de l'animal à sang chaud.

8. Utilisation suivant les revendications 1 et 4, dans laquelle l'ectoparasite est une puce et l'animal à sang chaud est un chien ou un chat.

9. Composition comprenant une quantité efficace du point de vue ovicide d'un composé choisi dans le groupe ayant la formule générale : 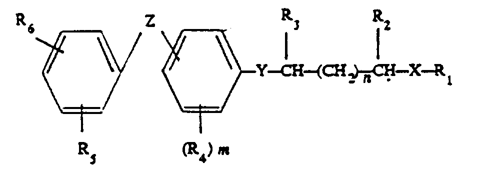 dans laquelle R₁ est l'un des groupes suivants : 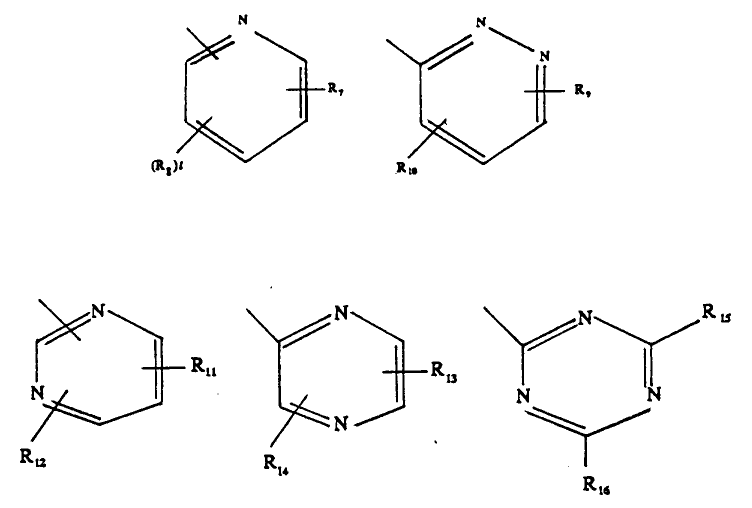 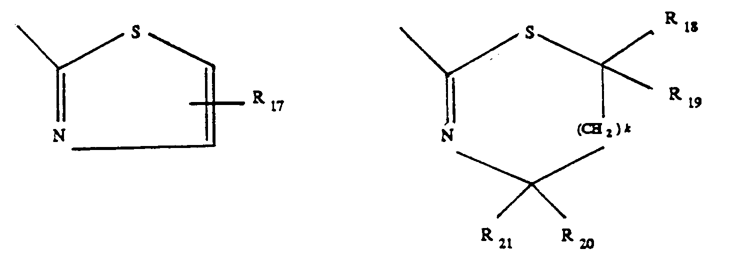 dans lesquels R₇, R₈, R₉, R₁₀, R₁₁, R₁₂, R₁₃, R₁₄, R₁₅, R₁₆ et R₁₇, identiques ou différents, sont chacun un atome d'hydrogène ou d'halogène, un groupe alcoxy en C₁₋₄, alkylthio en C₁₋₄, trifluorométhyle ou nitro ; R₁₈, R₁₉, R₂₀ et R₂₁, identiques ou différents, sont chacun un atome d'hydrogène ou un groupe méthyle, *k* est un entier de 0 à 1, et *l* est un entier de 0 à 3 ; R₂ et R₃, identiques ou différents, sont chacun un atome d'hydrogène ou d'halogène ou un groupe méthyle ; R₄ est un atome d'halogène ou un groupe méthyle ; R₅ et R₆, identiques ou différents, sont chacun un atome d'hydrogène ou d'halogène, un groupe haloalkyle en C₁₋₄, ou haloalcoxy en C₁₋₄ ; X, Y et Z, identiques ou différents, sont chacun un atome d'oxygène, de soufre ou un groupe méthylène, *m* est un entier de 0 à 4, et *n* est un entier de 0 à 2 et une quantité efficace du point de vue parasiticide d'un composé choisi dans le groupe consistant en avermectine, dérivés d'avermectine, milbémycine, dérivés de milbémycine, ivermectine, dérivés d'ivermectine, milbémycine oxime, dérivés de milbémycine oxime, moxidectine et dérivés de moxidectine, ou leurs mélanges.

10. Composition selon la revendication 9, comprenant de plus un matériau fournissant une distribution continue acceptable du point de vue pharmaceutique.

11. Composition suivant la revendication 10, dans laquelle le matériau fournissant une distribution continue comprend 6 parties de silicone élastomère pour 1 partie de polymère méthacrylique hydrophile.

12. Composition selon la revendication 10, dans laquelle les composés sont le pyriproxifen et la moxidectine.

13. Composition selon la revendication 9, comprenant de plus un conservateur et/ou un arôme.

14. Composition selon la revendication 9, comprenant de plus un gel.

15. Composition selon la revendication 9, comprenant de plus une vitamine.

16. Utilisation d'une composition selon la revendication 9 pour la préparation d'une composition pharmaceutique pour la lutte systémique contre les parasites, la composition étant adaptée à l'administration à un animal à sang chaud de telle sorte que le composé ovicide et le composé parasiticide soient absorbés dans le sang de l'animal et transmis aux parasites par ingestion ou contact avec le sang de l'animal.

17. Utilisation selon la revendication 16, dans laquelle la composition est administrée par un implant.

18. Utilisation selon la revendication 17, dans laquelle l'implant est une composition comprenant 6 parties de silicone élastomère pour 1 partie de polymère méthacrylique hydrophile.

19. Utilisation d'une composition pour la préparation d'une composition pharmaceutique pour la lutte systémique simultanée contre les ectoparasites et les endoparasites chez les animaux à sang chaud, la composition étant adaptée à l'administration, à un animal à sang chaud, de 0,0001 à 1000 mg de pyriproxifen par kg de poids corporel de l'animal et de 0,5 µg à 100 mg d'un composé choisi dans le groupe consistant en avermectine, dérivés d'avermectine, milbémycine, dérivés de milbémycine, ivermectine, dérivés d'ivermectine, milbémycine oxime, dérivés de milbémycine oxime, moxidectine et dérivés de moxidectine, ou leurs mélanges, par kg de poids corporel de l'animal.

20. Utilisation selon la revendication 19, dans laquelle la composition est adaptée à l'administration d'environ 0,2 à environ 50 mg de pyriproxifen par kg de poids corporel de l'animal et d'environ 1,25 à environ 50 µg de moxidectine par kg de poids corporel de l'animal.

21. Utilisation selon la revendication 19, dans laquelle l'animal à sang chaud est un chien.

22. Utilisation selon la revendication 19, dans laquelle la composition est administrée par un implant.

23. Supplément alimentaire comprenant une composition selon la revendication 9 ainsi qu'un conservateur, une vitamine, un sel minéral, et une huile de poisson Omega 3, ou leurs mélanges.

24. Supplément alimentaire selon la revendication 23, dans lequel les composés choisis sont le pyriproxifen et la milbémycine et dans lequel le pyriproxifen est présent en une quantité adaptée à l'administration d'une dose comprise entre environ 0,01 mg et environ 200 mg par kg de poids corporel de l'animal tandis que la milbémycine est présente en une quantité adaptée à l'administration d'une dose comprise entre environ 0,01 mg et environ 100 mg par kg de poids corporel de l'animal.

25. Supplément alimentaire selon la revendication 23, dans lequel les composés choisis sont le pyriproxifen et l'ivermectine et dans lequel le pyriproxifen est présent en une quantité adaptée à l'administration d'une dose comprise entre environ 0,01 mg et environ 200 mg par kg de poids corporel de l'animal tandis que l'ivermectine est présente en une quantité adaptée à l'administration d'une dose comprise entre environ 0,01 mg et environ 10 mg par kg de poids corporel de l'animal.

26. Supplément alimentaire selon la revendication 23, dans lequel les composés choisis sont le pyriproxifen et la milbémycine oxime et dans lequel le pyriproxifen est présent en une quantité adaptée à l'administration d'une dose comprise entre environ 0,01 mg et environ 200 mg par kg de poids corporel de l'animal tandis que la milbémycine oxime est présente en une quantité adaptée à l'administration d'une dose comprise entre environ 0,01 mg et environ 100 mg par kg de poids corporel de l'animal.

27. Supplément alimentaire selon la revendication 23, dans lequel les composés choisis sont le pyriproxifen et la moxidectine et dans lequel le pyriproxifen est présent en une quantité adaptée à l'administration d'une dose comprise entre environ 0,01 mg et environ 200 mg par kg de poids corporel de l'animal tandis que la moxidectine est présente en une quantitié adaptée à l'administration d'une dose comprise entre environ 0,01 µg et environ 100 µg par kg de poids corporel de l'animal.

## Claims

1. Application of a compound selected from among the group having the general formula: 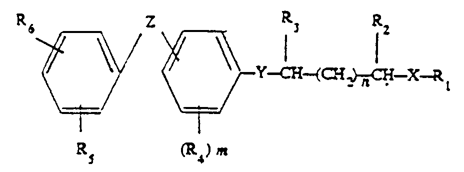 wherein R₁ is one of the following groups: 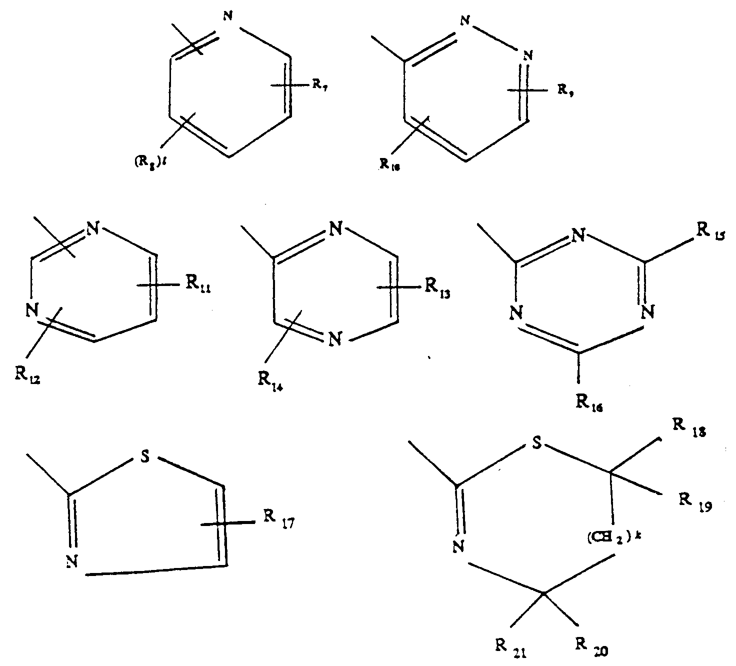 in which each of R₇,R₈,R₉,R₁₀,R₁₁,R₁₂,R₁₃,R₁₄,R₁₅,R₁₆ and R₁₇, which are identical or different, is a hydrogen atom or a halogen atom, a C₁-C₄ alkoxy group, a C₁-C₄ alkylthio group, a trifluoromethyl group or a nitro group; each of R₁₈, R₁₉, R₂₀, and R₂₁, which are identical or different, is a hydrogen atom or a methyl group, *k* is an integer from 0 to 1 and *l* is an integer from 0 to 3; each of R₂ and R₃, which are identical or different, is a hydrogen atom or a halogen atom or a methyl group; R₄ is a halogen atom or a methyl group; each of R₅ and R₆, which are identical or different, is a hydrogen atom or a halogen atom, a C₁-C₄ haloalkyl group or a C₁-C₄ haloalkoxy group; each of X, Y, and Z, which are identical or different, is an oxygen atom, a sulphur atom, or a methylene group, *m* is an integer from 0 to 4, and *n* is an integer from 0 to 2, for the preparation of a pharmaceutical composition intended for the systematic control of ectoparasites which attack warm-blooded animals.

2. Application according to Claim 1, in which the compound used has, as R₁, pyridine substituted at position 2.

3. Application according to Claim 2, characterised in that the compound used is a (4-phenoxyphenoxy)-ethoxy pyridine.

4. Application according to Claim 1, wherein the compound is 2-[1-methyl-2-(4-phenoxyphenoxy)-ethoxy] pyridine.

5. Application according to Claim 1, wherein the compound is suitable for administration of a dose of compound of between approximately 0.0001 mg and approximately 1000 mg per kg of body weight of the warm-blooded animal.

6. Application according to Claim 1, wherein the compound is suitable for administration of a dose of compound of between approximately 0.01 mg and 100 mg per kg of body weight of the warm-blooded animal.

7. Application according to Claim 1, in which the composition is suitable for administration of a dose of compound of between approximately 0.02 mg and approximately 50 mg per kg of body weight of the warm-blooded animal.

8. Application according to Claims 1 and 4, in which the ectoparasite is a flea and the warm-blooded animal is a dog or a cat.

9. Composition comprising an ovicidally effective quantity of a compound selected from the group having the general formula: 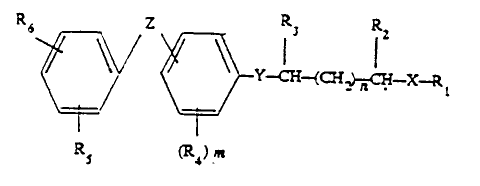 in which R₁ is one of the following groups: 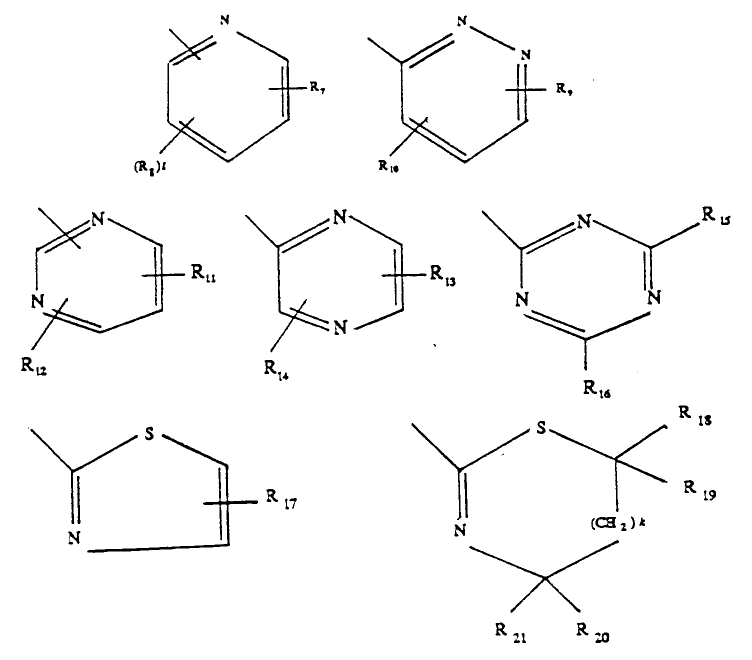 in which each of R₇,R₈,R₉,R₁₀,R₁₁,R₁₂,R₁₃,R₁₄,R₁₅,R₁₆ and R₁₇, which are identical or different, is a hydrogen atom or a halogen atom, a C₁-C₄ alkoxy group, a C₁-C₄ alkylthio group, a trifluoromethyl group or a nitro group; each of R₁₈, R₁₉, R₂₀, and R₂₁, which are identical or different, is a hydrogen atom or a methyl group, *k* is an integer from 0 to 1 and *l* is an integer from 0 to 3; each of R₂ and R₃, which are identical or different, is a hydrogen atom or a halogen atom or a methyl group; R₄ is a halogen atom or a methyl group; each of R₅ and R₆, which are identical or different, is a hydrogen atom or a halogen atom, a C₁-C₄ haloalkyl group or a C₁-C₄ haloalkoxy group; each of X, Y, and Z, which are identical or different, is an oxygen atom, a sulphur atom, or a methylene group, *m* is an integer from 0 to 4, and *n* is an integer from 0 to 2 and a parasiticidally active amount of a compound selected from the group consisting of avermectin, avermectin derivatives, milbemycin, milbemycin derivatives, ivermectin, ivermectin derivatives, milbemycin oxime, milbemycin oxime derivatives, moxidectin and moxidectin derivatives, or mixtures thereof.

10. Composition according to Claim 9, comprising, furthermore, a pharmaceutically acceptable sustained delivery material.

11. Composition according to Claim 10, in which the sustained delivery material comprises 6 parts of elastomer silicone for 1 part of hydrophillic methacrylic polymer.

12. Composition according to Claim 10, in which the compounds are pyriproxifen and moxidectin.

13. Composition according to Claim 9, furthermore comprising a preservative and/or a flavouring.

14. Composition according to Claim 9, furthermore comprising a gel.

15. Composition according to Claim 9, furthermore comprising a vitamin.

16. Application of a composition according to Claim 9 for the preparation of a pharmaceutical composition for the systematic control of parasites, the composition being suitable for adminstration to a warm-blooded animal in such a manner that the ovicidal composition and the parasiticidal composition are absorbed into the blood of the animal and transmitted to the parasites by ingestion or contact with the blood of the animal.

17. Application according to Claim 16, characterised in that the composition is administered by an implant.

18. Application according to Claim 17, in which the implant is a composition comprising 6 parts of elastomer silicone for 1 part of hydrophillic methacrylic polymer.

19. Application of a composition for the preparation of a pharmaceutical composition for the simultaneous systematic control of ectoparasites and endoparasites in warm-blooded animals, the composition being suitable for administration, to a warm-blooded animal, of 0.0001 to 1000 mg of pyriproxifen per kg of body weight of the animal and of from 0.5µg to 100 mg of a compound selected from among the group consisting of avermectin, avermectin derivatives, milbemycin, milbemycin derivatives, ivermectin, ivermectin derivatives, milbemicin oxime, milbemycin oxime derivatives, moxidectin and moxidectin derivatives, or mixtures thereof, by kg of body weight of the animal.

20. Application according to Claim 19, in which the composition is adapted to the application of approximately 0.2 to approximately 50 mg of pyriproxifen per kg of body weight of the animal and of approximately 1.25 to approximately 50 µg of moxidectin per kg of body weight of the animal.

21. Application according to Claim 19, in which the warm-blooded animal is a dog.

22. Application according to Claim 19, in which the compound is administered by an implant.

23. Food supplement comprising a composition according to Claim 9, as well as a preservative, a vitamin, a mineral salt, and an Omega 3 fish oil, or mixtures thereof.

24. Food supplement according to Claim 23 in which the selected compounds are pyriproxifen and milbemycin and in which the pyriproxifen is present in a quantity which is suitable for administration of a dose of between approximately 0.01 and approximately 200 mg per kg of body weight of the animal whereas the milbemycin is present in a quantity which is suitable for administration of a dose of between approximately 0.01 mg and approximately 100 mg per kg of body weight of the animal.

25. Food supplement according to Claim 23, in which the compound which are selected are pyriproxifen and ivermectin and in which the pyriproxifen is present in a quantity which is suitable for administration of a dose of between approximately 0.01 mg and approximately 200 mg per kg of body weight of the animal whereas the ivermectin is present in a quantity which is suitable for administration of a dose of between approximately 0.01 mg and approximately 10 mg by kg of body weight of the animal.

26. Food supplement according to Claim 23, in which the selected compounds are pyriproxifen and milbemycin oxime and in which the pyriproxifen is present in a quantity which is suitable for administration of a dose of between approximately 0.01 mg and approximately 200 mg per kg of the body weight of the animal whereas the milbemycin oxime is present in a quantity which is suitable for administration of a dose of between 0.01 mg and approximately 100 mg per kg of body weight of the animal.

27. Food supplement according to Claim 23, in which the selected compounds are pyriproxifen and moxidectin and in which the pyriproxifen is present in a quantity which is suitable for administration of a dose of between approximately 0.01 mg and approximately 200 mg per kg of body weight of the animal whereas the moxidectin is present in a quantity suitable for administration of a dose of between approximately 0.01 µg and approximately 100 µg per kg of body weight of the animal.

## Patentansprüche

1. Verwendung einer Verbindung, ausgewählt aus der Gruppe mit der allgemeinen Formel: 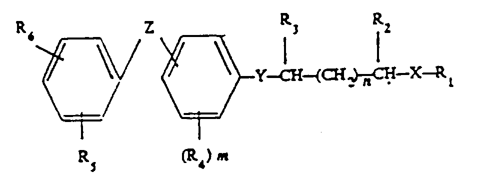 worin R₁ einer der folgenden Reste: 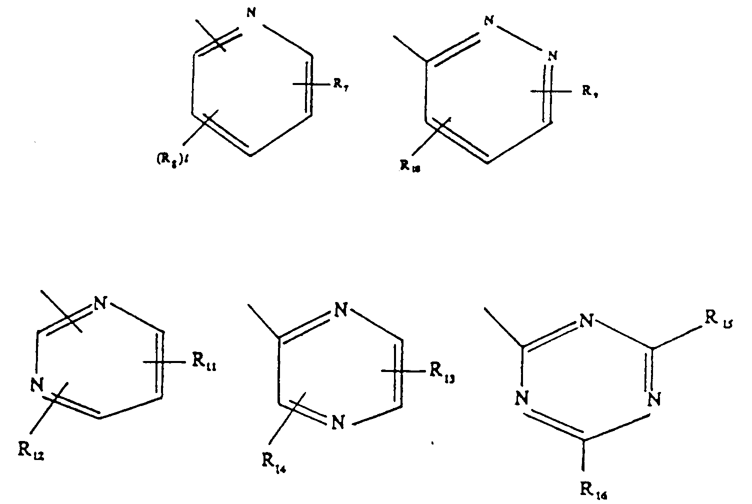 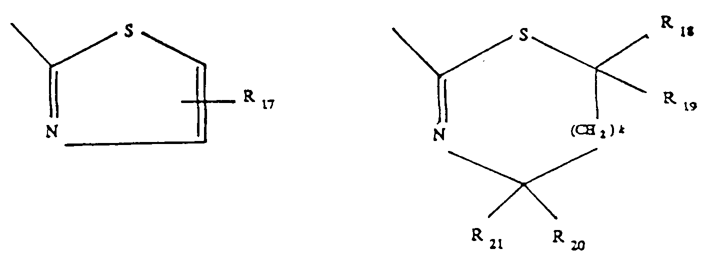 ist, worin R₇, R₈, R₉, R₁₀, R₁₁, R₁₂, R₁₃, R₁₄, R₁₅, R₁₆ und R₁₇, die gleich oder verschieden sind, jeweils ein Wasserstoff- oder ein Halogenatom, ein C₁₋₄-Alkoxyrest, ein C₁₋₄-Alkylthiorest, eine Trifluormethyl- oder Nitrogruppe sind; R₁₈, R₁₉, R₂₀ und R₂₁, die gleich oder verschieden sind, jeweils ein Wasserstoffatom oder eine Methylgruppe sind, *k* eine ganze Zahl von 0 bis 1 ist und *l* eine ganze Zahl von 0 bis 3 ist; R₂ und R₃, die gleich oder verschieden sind, jeweils ein Wasserstoff- oder Halogenatom oder eine Methylgruppe sind; R₄ ein Halogenatom oder eine Methylgruppe ist; R₅ und R₆, die gleich oder verschieden sind, jeweils ein Wasserstoff- oder Halogenatom, ein C₁₋₄-Halogenalkylrest oder ein C₁₋₄-Halogenalkoxyrest sind; X, Y und Z, die gleich oder verschieden sind, jeweils ein Sauerstoffatom, ein Schwefelatom oder eine Methylengruppe sind, *m* eine ganze Zahl von 0 bis 4 ist und *n* eine ganze Zahl von 0 bis 2 ist, zur Herstellung eines pharmazeutischen Präparats zur systemischen Bekämpfung von Ectoparasiten, die warmblütige Tiere angreifen.

2. Verwendung nach Anspruch 1, wobei bei der verwendeten Verbindung R₁ ein in Position 2 substituiertes Pyridin ist.

3. Verwendung nach Anspruch 2, wobei die verwendete Verbindung ein (4-Phenoxyphenoxy)ethoxypyridin ist.

4. Verwendung nach Anspruch 1, wobei die verwendete Verbindung 2-[1-Methyl-2-(4-phenoxyphenoxy)ethoxy]pyridin ist.

5. Verwendung nach Anspruch 1, wobei das Präparat der Verabreichung einer Dosis der Verbindung zwischen etwa 0,0001 mg und etwa 1000 mg pro kg Körpergewicht des warmblütigen Tieres angepaßt ist.

6. Verwendung nach Anspruch 1, wobei das Präparat der Verabreichung einer Dosis der Verbindung zwischen etwa 0,01 mg und etwa 100 mg pro kg Körpergewicht des warmblütigen Tieres angepaßt ist.

7. Verwendung nach Anspruch 1, wobei das Präparat der Verabreichung einer Dosis der Verbindung zwischen etwa 0,02 mg und etwa 50 mg pro kg Körpergewicht des warmblütigen Tieres angepaßt ist.

8. Verwendung nach den Ansprüchen 1 und 4, wobei der Ectoparasit ein Floh ist, und das warmblütige Tier ein Hund oder eine Katze ist.

9. Präparat, umfassend eine ovicid wirksame Menge einer Verbindung ausgewählt aus der Gruppe mit der allgemeinen Formel: 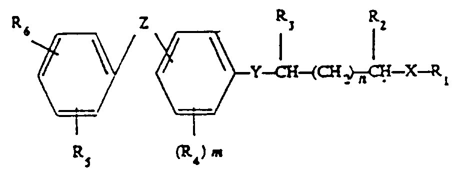 worin R₁ einer der folgenden Reste 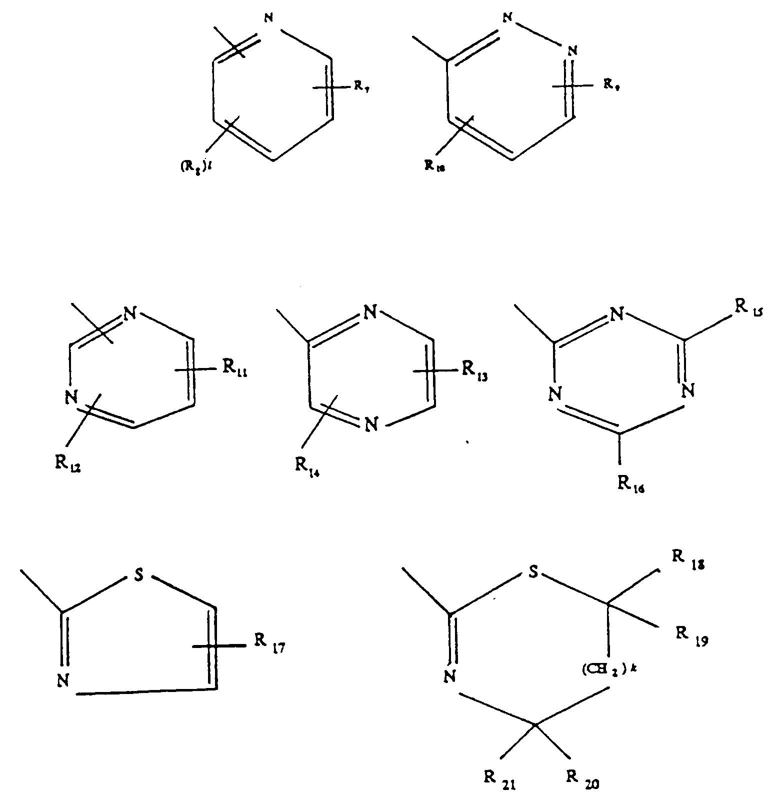 ist, worin R₇, R₈, R₉, R₁₀, R₁₁, R₁₂, R₁₃, R₁₄, R₁₅, R₁₆ und R₁₇, die gleich oder verschieden sind, jeweils ein Wasserstoff- oder ein Halogenatom, ein C₁₋₄-Alkoxyrest, ein C₁₋₄-Alkylthiorest, eine Trifluormethylgruppe oder eine Nitrogruppe sind; R₁₈, R₁₉, R₂₀ und R₂₁, die gleich oder verschieden sind, jeweils ein Wasserstoffatom oder eine Methylgruppe sind, *k* eine ganze Zahl von 0 bis 1 ist und *l* eine ganze Zahl von 0 bis 3 ist; R₂ und R₃, die gleich oder verschieden sind, jeweils ein Wasserstoff- oder ein Halogenatom oder eine Methylgruppe sind; R₄ ein Halogenatom oder ein Methylgruppe ist; R₅ und R₆, die gleich oder verschieden sind, jeweils ein Wasserstoff- oder ein Halogenatom, ein C₁₋₄-Halogenalkylrest oder ein C₁₋₄-Halogenalkoxyrest sind; X, Y und Z, die gleich oder verschieden sind, jeweils ein Sauerstoffatom, ein Schwefelatom oder eine Methylengruppe sind, *m* eine ganze Zahl von 0 bis 4 ist und *n* eine ganze Zahl von 0 bis 2 ist, und eine im Hinblick auf die Parasitenabtötung wirksame Menge einer Verbindung, ausgewählt aus der Gruppe bestehend aus Avermectin, Avermectinderivaten, Milbemycin, Milbemycinderivaten, Ivermectin, Ivermectinderivaten, Milbemycinoxim, Milbemycinoximderivaten, Moxidectin und Moxidectinderivaten oder ihren Gemischen.

10. Präparat nach Anspruch 9, umfassend weiterhin eine Substanz, die eine kontinuierliche, pharmazeutisch verträgliche Verteilung liefert.

11. Präparat nach Anspruch 10, worin die Substanz, die eine kontinuierliche Verteilung liefert, sechs Teile Silicon-elastomeres auf einen Teil hydrophiles Methacrylsäurepolymeres umfaßt.

12. Präparat nach Anspruch 10, worin die Verbindungen Pyriproxifen und Moxidectin sind.

13. Präparat nach Anspruch 9, umfassend weiterhin einen Konservierungsstoff und/oder einen aromagebenden Stoff.

14. Präparat nach Anspruch 9, umfassend weiterhin ein Gel.

15. Präparat nach Anspruch 9, umfassend weiterhin ein Vitamin.

16. Verwendung eines Präparats nach Anspruch 9 zur Herstellung eines pharmazeutischen Präparats zur systemischen Bekämpfung von Parasiten, wobei das Präparat der Verabreichung an ein warmblütiges Tier so angepaßt ist, daß die ovicide Verbindung und die parasitentötende Verbindung in das Blut des Tieres absorbiert werden und an die Parasiten durch Aufnahme oder Kontakt mit dem Blut des Tieres übertragen werden.

17. Verwendung nach Anspruch 16, wobei das Präparat über ein Implantat verabreicht wird.

18. Verwendung nach Anspruch 17, wobei das Implantat ein Präparat, umfassend sechs Teile Siliconelastomeres auf einen Teil hydrophiles Methacrylsäurepolymeres, ist.

19. Verwendung eines Präparats zur Herstellung eines pharmazeutischen Präparats zur gleichzeitigen systemischen Bekämpfung von Ectoparasiten und Endoparasiten bei warmblütigen Tieren, wobei das Präparat der Verabreichung von 0,0001 bis 1000 mg Pyriproxifen pro kg Körpergewicht des Tieres und 0,5 µg bis 100 mg einer Verbindung, ausgewählt aus der Gruppe bestehend aus Avermectin, Avermectinderivaten, Milbemycin, Milbemycinderivaten, Ivermectin, Ivermectinderivaten, Milbemycinoxim, Milbemycinoximderivaten, Moxidectin und Moxidectinderivaten oder ihren Gemischen, pro kg Körpergewicht des Tieres an ein warmblütiges Tier angepaßt ist.

20. Verwendung nach Anspruch 19, wobei das Präparat der Verabreichung von etwa 0,2 bis etwa 50 mg Pyriproxifen pro kg Körpergewicht des Tieres und etwa 1,25 bis etwa 50 µg Moxidectin pro kg Körpergewicht des Tieres angepaßt ist.

21. Verwendung nach Anspruch 19, wobei das warmblütige Tier ein Hund ist.

22. Verwendung nach Anspruch 19, wobei das Präparat durch ein Implantat verabreicht wird.

23. Futtersupplement, umfassend ein Präparat nach Anspruch 9 sowie einen Konservierungsstoff, ein Vitamin, ein Mineralsalz, ein Omega-3-Fischöl oder Gemische davon.

24. Futtersupplement nach Anspruch 23, wobei die ausgewählten Verbindungen Pyriproxifen und Milbemycin sind und wobei das Pyriproxifen in einer Menge, die der Verabreichung einer Dosis zwischen etwa 0,01 mg und etwa 200 mg pro kg Körpergewicht des Tieres angepaßt ist, vorhanden ist, während das Milbemycin in einer Menge, die der Verabreichung einer Dosis zwischen etwa 0,01 mg und etwa 100 mg pro kg Körpergewicht des Tieres angepaßt ist, vorhanden ist.

25. Futtersupplement nach Anspruch 23, worin die gewählten Verbindungen Pyriproxifen und Ivermectin sind und worin das Pyriproxifen in einer Menge, die der Verabreichung einer Dosis zwischen etwa 0,01 mg und etwa 200 mg pro kg Körpergewicht des Tieres angepaßt ist, vorhanden ist, während das Ivermectin in einer Menge, die der Verabreichung einer Dosis zwischen etwa 0,01 mg und etwa 10 mg pro kg Körpergewicht des Tieres angepaßt ist, vorhanden ist.

26. Futtersupplement nach Anspruch 23, worin die gewählten Verbindungen Pyriproxifen und Milbemycinoxim sind und worin das Pyriproxifen in einer Menge, die der Verabreichung einer Dosis zwischen etwa 0,01 mg und etwa 200 mg pro kg Körpergewicht des Tieres angepaßt ist, vorhanden ist, während das Milbemycinoxim in einer Menge, die der Verabreichung einer Dosis zwischen etwa 0,01 mg und etwa 100 mg pro kg Körpergewicht des Tieres angepaßt ist, vorhanden ist.

27. Futtersupplement nach Anspruch 23, worin die gewählten Verbindungen Pyriproxifen und Moxidectin sind und worin das Pyriproxifen in einer Menge, die der Verabreichung einer Dosis zwischen etwa 0,01 mg und etwa 200 mg pro kg Körpergewicht des Tieres angepaßt ist, vorhanden ist, während das Moxidectin in einer Menge, die der Verabreichung einer Dosis zwischen etwa 0,01 µg und etwa 100 µg pro kg Körpergewicht des Tieres angepaßt ist, vorhanden ist.
